# EUROPEAN PATENT APPLICATION

(11) **EP 1 666 606 A1**
(43) Date of publication of application: **07.06.2006**
(21) Application number: 04772285.5
(22) Date of filing: 20.08.2004
(51) Int. Cl.: C12Q 1/26, C12Q 1/48, C12N 15/09, A61K 45/00, A61P 29/00

(54) **METHOD FOR DETECTING REGULATORY EFFECT ON CONTROLLING SYNOVIOLIN ACTIVITY**

(30) Priority: 20.08.2003 JP 2003295964; 20.08.2003 JP 2003295951
(71) Applicant: Locomogene, Inc., Tokyo 105-0001 (JP); Eisai Co., Ltd., Tokyo 112-8088 (JP)
(72) Inventor: NAKAJIMA, Toshihiro, Kohoku Garden Hills A-503, Yokohama-shi, Kanagawa 2240001 (JP); AMANO, Tetsuya, Kawasaki-shi, Kanagawa 2140005 (JP); ZHANG, Lei, Tokyo 1700003 (JP); IKEDA, Rie, Kawasaki-shi, Kanagawa 2160015 (JP); YAMASAKI, Satoshi, Ishikawazaka Mansion 305, Yokohama-shi, Kanagawa 2250003 (JP); YAGISHITA, Naoko, Yokohama-shi, Kanagawa 2340053 (JP); YOKOHAMA, Hiromitsu, Eisa Co., Ltd. Res. Lab., Tsukuba-shi, Ibaraki 3002635 (JP); KOGUSHI, Motoji, Eisa Co., Ltd. Res. Lab., Tsukuba-shi, Ibaraki 3002635 (JP)
(74) Representative: Harding, Charles Thomas
(86) International application number: PCT/JP2004/012329
(87) International publication number: WO 2005/019472

(57) **Abstract**

The present invention provides methods for evaluating the regulation on prolyl 4-hydroxylase activity. The present invention also provides screening methods using the evaluation methods. Furthermore, the present invention provides methods of screening for compounds that are useful for treating or preventing fibrosis or rheumatoid arthritis.

The present invention relates to methods for detecting regulatory activity on the synoviolin ubiquitination of prolyl 4-hydroxylase α subunit (P4HA1). It also relates to methods of screening for compounds with regulatory effect on the ubiquitination activity of synoviolin on P4HA1 based on these methods. Compounds discovered in the screening are useful for treating and/or preventing diseases caused by abnormalities in the prolyl 4-hydroxylase activity, such as fibrosis and rheumatoid arthritis.

## Description

### Technical Field

The present invention relates to methods for detecting regulatory effect on synoviolin activity.

Furthermore, the present invention relates to methods of screening for compounds that are useful for treating or preventing fibrosis and/or rheumatism.

### Background Art

Synoviolin is a protein which was discovered as a membrane protein present in synovial cells derived from patients with rheumatism (see WO 02/052007). Researches using genetically modified animals revealed that this factor directly participates in osteal/articular developments and the onset of arthrosis. Consequently, synoviolin is considered to be a protein with activities that contribute to normal osteogenesis or normal development of extremities. Protein structure prediction systems revealed the presence of a RING finger motif in synoviolin. This motif is known to exist among E3 ubiquitin protein ligases, which are involved in protein degradation. In fact, it has been proven that synoviolin has an autoubiquitination activity which is a feature of the RING finger-type E3 ubiquitin protein ligases (see WO 02/052007).

Searching for synoviolin-binding factors is an effective way to elucidate the function of synoviolin in osteal/articular developments and arthrosis, i.e., the type of intracellular signaling pathway synoviolin participates in. Particularly, identification of substrate proteins for synoviolin is an important task in understanding the intracellular signaling pathway in which synoviolin participates.

The identification of synoviolin-binding proteins, in particular, substrate proteins of synoviolin, is useful for clarifying the function of synoviolin in osteal/articular developments and arthrosis and in developing novel methods for arthrosis diagnosis and treatment. However, substrates of synoviolin have not yet been identified. Consequently, substances that affect the interaction between synoviolin and its substrates are yet unrevealed.

Meanwhile, collagen is an important constitutive component in the living body and has the following characteristics:
- is a main component of connective tissue;
- has one or more domains with a triple helix structure;
- forms a supramolecular assembly; and
- constitutes an extracellular matrix.

One of the principal roles of collagen is forming a tissue structure to serve as an anchorage for cells. Collagen also affects the migration, differentiation, proliferation, and metabolic functions of cells. Fibroblasts are the major collagen-producing cells. Initially, preprocollagen is synthesized in a cell and converted into procollagen in the rough-surfaced endoplasmic reticulum. Propeptides are linked to the N- and C-termini of a procollagen. The procollagen is converted into tropocollagen through procollagen peptidase cleavage of the propeptides immediately before its secretion from the cell. The secreted tropocollagen is a fundamental component of collagen fiber. The secreted tropocollagens are crosslinked with each other to form collagen fiber bundles.

Prolyl 4-hydroxylase is an essential enzyme in collagen synthesis. The structure of prolyl 4-hydroxylase is already known. Specifically, prolyl 4-hydroxylase has a tetramer structure composed of an assembly of two α subunits and two β subunits. It is thought that the α subunits have a function of catalyzing the hydroxylation reaction of proline, and the β subunits have a disulfide isomerase activity. The α subunit that constitutes prolyl 4-hydroxylase is referred to as P4HA1. Prolyl 4-hydroxylase hydroxylates a proline residue on the -X-Pro-Gly sequence of procollagen which thereby forms a stable collagen triple helix structure (see Pihlajanicmi, T., *et al.,* J. Hepatol., 1991, 13, S2-7).

Furthermore, prolyl 4-hydroxylase has been reported to have a function of preventing the secretion of unhydroxylated procollagen from endoplasmic reticulum by binding therewith (see Walmsley, A. R., *et.al.,* J. Biolog. Chem., 1999, 274 (21), p14884-14892). The unhydroxylated procollagen is an immature procollagen. Consequently, prolyl 4-hydroxylase is considered to serve as quality control for collagen in the living body.

While collagen is an important constitutive element of the body, it causes pathological biological reactions such as fibrosis. For example, accumulation of collagen in tissues such as lung, liver, or kidney causes fibrosis of the organ. In rheumatism and osteoarthrosis deformans, abnormalities in collagen-containing extracellular matrices of the cartilage and synovial cells in the joints were observed (see Masataka KUWANA, Molecular Medicine, 2001, Vol. 38(8), p900-907).

Consequently, there is a possibility that these diseases can be treated by controlling collagen production. For example, it has been reported that these diseases may be treated through regulation of collagen production by inhibiting the activity of stress protein HSP47 (heat shock protein 47) (see Kivirikko KI., Ann Med., 1993, Apr; 25 (2): 113-26). Prolyl 4-hydroxylase and collagen production by this enzyme will become an important target in the development of diagnosis and treatment methods for fibrosis and arthritis. However, the mechanism of regulating the activity of prolyl 4-hydroxylase has not yet been clarified.

Rheumatoid arthritis (hereinafter referred to as RA or rheumatism) is a general chronic inflammatory disease in which abnormal proliferation of the articular synovial membrane tissues is observed. Synoviocyte (synovial cell) is a fibroblast-like cell that constitutes one to six adepithelial layers in the articular synovial membrane and is believed to supply proteoglycan and hyaluronic acid to the synovial fluid. In the joints of RA patients, symptoms such as proliferation of the synovial membrane tissue (i.e., a resulting multilayer structure), and invasion of synovial cells into another tissue are observed. An autoantibody against the Fc region of an autologous IgG exists in the sera of RA patients. Thus, rheumatism is considered as an autoimmune disease, but the cause thereof has not yet been elucidated.

### Disclosure of the Invention

An objective of the present invention is to elucidate the regulatory mechanism of prolyl 4-hydroxylase activity. Another objective of the present invention is to provide methods for evaluating the regulation of prolyl 4-hydroxylase activity. Yet another objective of the present invention is to provide methods of screening for compounds that have such a function using the evaluation methods. A further objective of the present invention is to provide methods of screening for compounds that are useful for the treatment or prevention of rheumatoid arthritis or fibrosis.

The present inventors used a yeast two-hybrid method in the screening for identification of synoviolin-binding proteins. Synoviolin was used as bait in the screening of a human cartilage-derived cDNA library by a yeast two-hybrid system (Clontech, MATCHMAKER Two-Hybrid System). As a result, prolyl 4-hydroxylase α subunit (P4HA1) was obtained. Next, the binding between P4HA1 and synoviolin was verified by a GST pulldown analysis.

The present inventors further revealed that P4HA1 is ubiquitinated by the ubiquitin ligase activity of synoviolin. That is, P4HA1 is verified to be a substrate polypeptide of synoviolin ubiquitin ligase (E3). Synoviolin induces the proteasome degradation of P4HA1 via ubiquitination of P4HA1. Prolyl 4-hydroxylase is a key enzyme in collagen synthesis. Consequently, there is a possibility that synoviolin participates in individual growth, osteal/articular developments, and onset of arthrosis through regulation of the prolyl 4-hydroxylase activity.

Based on these findings, the present inventors established methods for evaluating interference on the interaction between synoviolin and P4HA1. They also discovered that it is possible to use these methods to screen for a substance having such an effect. The present invention was achieved based on these findings. Specifically, the present invention relates to the following methods for detecting the regulatory activity of a test compound on the ubiquitination effect of synoviolin. Alternatively, the present invention relates to methods of screening for compounds with regulatory activity on the ubiquitination effect of synoviolin by utilizing these methods.
[1] A method for detecting regulatory activity of a test compound on the ubiquitination effect of synoviolin, comprising the steps of:
   a) incubating synoviolin or a homolog thereof and a substrate in the presence of a test compound under enabling conditions for substrate ubiquitination,
   a') contacting either (i) a substrate or (ii) synoviolin or a homolog thereof with a test compound and incubating with the other of (i) and (ii) under enabling conditions for substrate ubiquitination, or
   a") incubating synoviolin or a homolog thereof and a substrate under enabling conditions for substrate ubiquitination and contacting them with a test compound;
   b) measuring level of substrate ubiquitination after any one of steps a), a'), and a"); and
   c) detecting regulatory activity of the test compound on the synoviolin ubiquitination of the substrate when the substrate ubiquitination level differs from a ubiquitination level measured in the absence of the test compound,
   wherein the substrate is prolyl 4-hydroxylase α subunit or a homolog thereof.
[2] The method of [1], wherein the enabling conditions for substrate ubiquitination are provided by coexistence of the following components:
   i) a ubiquitin activating enzyme;
   ii) a ubiquitin transferase;
   iii) ubiquitin; and
   iv) adenosine triphosphate.
[3] The method of [1], wherein the enabling conditions for substrate ubiquitination are provided by ubiquitinating the substrate in a cell expressing the substrate and the synoviolin or a homolog thereof.
[4] The method of [1], wherein the ubiquitination level of the substrate is measured using any one of the following levels as an index:
   A) level of ubiquitinated substrate;
   B) level of unubiquitinated substrate; and
   C) level of prolyl 4-hydroxylase α subunit bioactivity.
[5] The method of [1], wherein the prolyl 4-hydroxylase α subunit or a homolog thereof is any one of the following polypeptides (a) to (e):
   (a) a polypeptide encoded by the nucleotide sequence of SEQ ID NO: 1;
   (b) a polypeptide comprising the amino acid sequence of SEQ ID NO: 2;
   (c) a polypeptide that comprises an amino acid sequence with a substitution, deletion, insertion, and/or addition of one or plural amino acids in the amino acid sequence of SEQ ID NO: 2 and which can be ubiquitinated by synoviolin;
   (d) a polypeptide that comprises an amino acid sequence encoded by a polynucleotide hybridizing under stringent conditions with a DNA comprising the nucleotide sequence of SEQ ID NO: 1 and which can be ubiquitinated by synoviolin; and
   (e) a polypeptide that comprises an amino acid sequence having a homology of 70% or more with the amino acid sequence of SEQ ID NO: 2 and which can be ubiquitinated by synoviolin.
[6] The method of [1], wherein the synoviolin or a homolog thereof is any one of the following polypeptides (A) to (E):
   (A) a polypeptide encoded by the nucleotide sequence of SEQ ID NO: 3;
   (B) a polypeptide comprising the amino acid sequence of SEQ ID NO: 4;
   (C) a polypeptide that comprises an amino acid sequence with a substitution, deletion, insertion, and/or addition of one or plural amino acids in the amino acid sequence of SEQ ID NO: 4 and which has an activity of ubiquitinating a polypeptide comprising the amino acid sequence of SEQ ID NO: 2;
   (D) a polypeptide which hybridizes under stringent conditions with a DNA comprising the nucleotide sequence of SEQ ID NO: 3 and that has an activity of ubiquitinating a polypeptide comprising the amino acid sequence of SEQ ID NO: 2; and
   (E) a polypeptide which comprises an amino acid sequence having a homology of 70% or more with the amino acid sequence of SEQ ID NO: 4 and has an activity of ubiquitinating a polypeptide comprising the amino acid sequence of SEQ ID NO: 2.
[7] A method of screening for a test compound having regulatory activity on the ubiquitination effect of synoviolin, comprising the steps of:
   a) detecting regulatory activity of a test compound on the ubiquitination effect of synoviolin by the method of any one of [1] to [6]; and
   b) selecting a test compound with a different substrate ubiquitination level when compared with a control.
[8] An agent for regulating the synoviolin ubiquitination of a substrate, comprising a compound obtainable by the method of [7] as an active ingredient.
[9] A kit for detecting regulatory activity on the ubiquitination effect of synoviolin, comprising the following components:
   (1) synoviolin or a homolog thereof; and
   (2) prolyl 4-hydroxylase α subunit or a homolog thereof.
[10] The kit of [9], further comprising the following components:
   (3) a ubiquitin activating enzyme;
   (4) a ubiquitin transferase;
   (5) ubiquitin; and
   (6) adenosine triphosphate.
[11] A kit for detecting regulatory activity on the ubiquitination effect of synoviolin, comprising a cell expressing synoviolin or a homolog thereof, and prolyl 4-hydroxylase α subunit or a homolog thereof; and a means for measuring ubiquitination level of the prolyl 4-hydroxylase α subunit or a homolog thereof.
[12] A method for detecting regulatory activity of a test compound on the ubiquitination effect of synoviolin, comprising the steps of:
   a) incubating in the presence of a test compound under enabling conditions for binding of the synoviolin or a homolog thereof and the substrate,
   a') contacting either (i) a substrate or (ii) synoviolin or a homolog thereof with a test compound and incubating under enabling conditions for binding of the synoviolin and the substrate, or
   a") incubating under enabling conditions for binding of the substrate and the synoviolin or a homolog thereof and contacting them with a test compound;
   b) measuring the level of binding between the substrate and the synoviolin or a homolog thereof after any one of steps a), a'), and a"); and
   c) detecting regulatory activity of the test compound on the synoviolin ubiquitination of the substrate when the level of binding between the substrate and the synoviolin or a homolog thereof differs from a binding level measured in the absence of the test compound,
   wherein the substrate is prolyl 4-hydroxylase α subunit or a homolog thereof.
[13] The method of [12], wherein either the synoviolin or the substrate is bound to a solid phase or comprises a label capable of binding to a solid phase.
[14] The method of [12], wherein the prolyl 4-hydroxylase α subunit or a homolog thereof is any one of the following polypeptides (a) to (e):
   (a) a polypeptide encoded by the nucleotide sequence of SEQ ID NO: 1;
   (b) a polypeptide comprising the amino acid sequence of SEQ ID NO: 2;
   (c) a polypeptide which comprises an amino acid sequence with a substitution, deletion, insertion, and/or addition of one or plural amino acids in the amino acid sequence of SEQ ID NO: 2 and can bind to synoviolin;
   (d) a polypeptide which comprises an amino acid sequence encoded by a polynucleotide hybridizing under stringent conditions with a DNA comprising the nucleotide sequence of SEQ ID NO: 1 and can bind to synoviolin; and
   (e) a polypeptide which comprises an amino acid sequence having a homology of 70% or more with the amino acid sequence of SEQ ID NO: 2 and can bind to synoviolin.
[15] The method of [12], wherein the synoviolin or a homolog thereof is any one of the following polypeptides (A) to (E):
   (A) a polypeptide encoded by the nucleotide sequence of SEQ ID NO: 3;
   (B) a polypeptide comprising the amino acid sequence of SEQ ID NO: 4;
   (C) a polypeptide which comprises an amino acid sequence with a substitution, deletion, insertion, and/or addition of one or plural amino acids in the amino acid sequence of SEQ ID NO: 4 and can bind to a polypeptide comprising the amino acid sequence of SEQ ID NO: 2;
   (D) a polypeptide which hybridizes under stringent conditions with a DNA comprising the nucleotide sequence of SEQ ID NO: 3 and that can bind to a polypeptide comprising the amino acid sequence of SEQ ID NO: 2; and
   (E) a polypeptide which comprises an amino acid sequence having a homology of 70% or more with the amino acid sequence of SEQ ID NO: 4 and can bind to a polypeptide comprising the amino acid sequence of SEQ ID NO: 2.
[16] A method of screening for a test compound having regulatory activity on the ubiquitination effect of synoviolin, comprising the steps of:
   a) detecting regulatory activity of a test compound on the ubiquitination effect of synoviolin by the method of any one of [12] to [15]; and
   b) selecting a test compound with a different level of binding between synoviolin and the substrate when compared with a control.
[17] An agent for regulating the synoviolin ubiquitination of a substrate, comprising a compound obtainable by the method of [16] as an active ingredient.
   The present inventors also discovered that it is possible to screen for useful compounds for the treatment or prevention of fibrosis and thereby completed the present invention. Specifically, the present invention relates to the following screening methods, kits therefor, and pharmaceutical compositions for treating and/or preventing fibrosis which comprise a compound obtainable by these methods as an active ingredient.
[18] A method of screening for a compound for treatment or prevention of fibrosis, comprising the steps of:
   a) incubating synoviolin or a homolog thereof and a substrate in the presence of a test compound under enabling conditions for substrate ubiquitination,
   a') contacting either (i) a substrate or (ii) synoviolin or a homolog thereof with a test compound and incubating with the other of (i) and (ii) under enabling conditions for substrate ubiquitination, or
   a") incubating a substrate with synoviolin or a homolog thereof under enabling conditions for substrate ubiquitination, and then contacting them with a test compound;
   b) measuring level of substrate ubiquitination after any one of steps a), a'), and a"); and
   c) selecting a test compound with a substrate ubiquitination level lower than a ubiquitination level measured in the absence of the test compound as a compound for treatment or prevention of fibrosis,
      wherein the substrate is prolyl 4-hydroxylase α subunit or a homolog thereof.
[19] The method of [18], wherein the enabling conditions for substrate ubiquitination are provided by coexistence of the following ingredients:
   i) a ubiquitin activating enzyme;
   ii) a ubiquitin transferase;
   iii) ubiquitin; and
   iv) adenosine triphosphate.
[20] The method of [18], wherein the enabling conditions for substrate ubiquitination are provided by ubiquitinating the substrate in a cell expressing the substrate and the synoviolin or a homolog thereof.
[21] The method of [18], wherein the ubiquitination level of the substrate is measured using any one of the following levels as an index:
   A) level of ubiquitinated substrate;
   B) level of unubiquitinated substrate; and
   C) level of prolyl 4-hydroxylase α subunit bioactivity.
[22] The method of [18], wherein the prolyl 4-hydroxylase α subunit or a homolog thereof is any one of the following polypeptides (a) to (e):
   (a) a polypeptide encoded by the nucleotide sequence of SEQ ID NO: 1;
   (b) a polypeptide comprising the amino acid sequence of SEQ ID NO: 2;
   (c) a polypeptide which comprises an amino acid sequence with a substitution, deletion, insertion, and/or addition of one or plural amino acids in the amino acid sequence of SEQ ID NO: 2 and can be ubiquitinated by synoviolin;
   (d) a polypeptide which comprises an amino acid sequence encoded by a polynucleotide hybridizing under stringent conditions with a DNA comprising the nucleotide sequence of SEQ ID NO: 1 and can be ubiquitinated by synoviolin; and
   (e) a polypeptide which comprises an amino acid sequence having a homology of 70% or more with the amino acid sequence of SEQ ID NO: 2 and can be ubiquitinated by synoviolin.
[23] The method of [18], wherein the synoviolin or a homolog thereof is any one of the following polypeptides (A) to (E):
   (A) a polypeptide encoded by the nucleotide sequence of SEQ ID NO: 3;
   (B) a polypeptide comprising the amino acid sequence of SEQ ID NO: 4;
   (C) a polypeptide which comprises an amino acid sequence with a substitution, deletion, insertion, and/or addition of one or plural amino acids in the amino acid sequence of SEQ ID NO: 4 and has an activity of ubiquitinating a polypeptide comprising the amino acid sequence of SEQ ID NO: 2;
   (D) a polypeptide which hybridizes under stringent conditions with a DNA comprising the nucleotide sequence of SEQ ID NO: 3 and that has an activity of ubiquitinating a polypeptide comprising the amino acid sequence of SEQ ID NO: 2; and
   (E) a polypeptide which comprises an amino acid sequence having a homology of 70% or more with the amino acid sequence of SEQ ID NO: 4 and has an activity of ubiquitinating a polypeptide comprising the amino acid sequence of SEQ ID NO: 2.
[24] A pharmaceutical composition for treating and/or preventing fibrosis, comprising a compound selectable by the method of any one of [18] to [23] as an active ingredient.
[25] A kit for screening for a compound for treatment or prevention of fibrosis, comprising the following components:
   (1) synoviolin or a homolog thereof; and
   (2) prolyl 4-hydroxylase α subunit or a homolog thereof.
[26] The kit of [25], further comprising the following components:
   (3) a ubiquitin activating enzyme;
   (4) a ubiquitin transferase;
   (5) ubiquitin; and
   (6) adenosine triphosphate.
[27] A kit for screening for a test compound for treatment or prevention of fibrosis, comprising a cell expressing synoviolin or a homolog thereof, and prolyl 4-hydroxylase α subunit or a homolog thereof; and a means for measuring the ubiquitination level of the prolyl 4-hydroxylase α subunit or a homolog thereof.
[28] A method of screening for a compound for treatment or prevention of fibrosis, comprising the steps of:
   a) incubating in the presence of a test compound under enabling conditions for binding of the synoviolin or a homolog thereof and the substrate,
   a') contacting either (i) a substrate or (ii) synoviolin or a homolog thereof with a test compound and incubating under enabling conditions for binding of the synoviolin and the substrate, or
   a") incubating under enabling conditions for binding of the substrate and the synoviolin or a homolog thereof and contacting them with a test compound;
   b) measuring the level of binding between the substrate and synoviolin or a homolog thereof after any one of steps a), a'), and a"); and
   c) selecting a test compound with a binding level between the substrate and the synoviolin or a homolog thereof lower than a binding level measured in the absence of the test compound as a compound for treatment or prevention of fibrosis,
   wherein the substrate is prolyl 4-hydroxylase α subunit or a homolog thereof.
[29] The method of [28], wherein either the synoviolin or the substrate is bound to a solid phase or comprises a label capable of binding to a solid phase.
[30] The method of [28], wherein the prolyl 4-hydroxylase α subunit or a homolog thereof is any one of the following polypeptides (a) to (e):
   (a) a polypeptide encoded by the nucleotide sequence of SEQ ID NO: 1;
   (b) a polypeptide comprising the amino acid sequence of SEQ ID NO: 2;
   (c) a polypeptide which comprises an amino acid sequence with a substitution, deletion, insertion, and/or addition of one or plural amino acids in the amino acid sequence of SEQ ID NO: 2 and can bind to synoviolin;
   (d) a polypeptide which comprises an amino acid sequence encoded by a polynucleotide hybridizing under stringent conditions with a DNA comprising the nucleotide sequence of SEQ ID NO: 1 and can bind to synoviolin; and
   (e) a polypeptide which comprises an amino acid sequence having a homology of 70% or more with the amino acid sequence of SEQ ID NO: 2 and can bind to synoviolin.
[31] The method of [28], wherein the synoviolin or a homolog thereof is any one of the following polypeptides (A) to (E):
   (A) a polypeptide encoded by the nucleotide sequence of SEQ ID NO: 3;
   (B) a polypeptide comprising the amino acid sequence of SEQ ID NO: 4;
   (C) a polypeptide which comprises an amino acid sequence with a substitution, deletion, insertion, and/or addition of one or plural amino acids in the amino acid sequence of SEQ ID NO: 4 and can bind to a polypeptide comprising the amino acid sequence of SEQ ID NO: 2;
   (D) a polypeptide which hybridizes under stringent conditions with a DNA comprising the nucleotide sequence of SEQ ID NO: 3 and that can bind to a polypeptide comprising the amino acid sequence of SEQ ID NO: 2, and
   (E) a polypeptide which comprises an amino acid sequence having a homology of 70% or more with the amino acid sequence of SEQ ID NO: 4 and can bind to a polypeptide comprising the amino acid sequence of SEQ ID NO: 2.
[32] A pharmaceutical composition for treating and/or preventing fibrosis, comprising a compound selectable by the method of any one of [28] to [31] as an active ingredient.
   The present inventors also discovered that it is possible to screen for useful compounds for the treatment or prevention of rheumatoid arthritis and thereby completed the present invention. Specifically, the present invention relates to the following screening methods, kits therefor, and pharmaceutical compositions for treating and/or preventing chronic rheumatoid arthritis which comprise a compound obtainable by these methods as an active ingredient.
[33] A method of screening for a compound for treatment or prevention of rheumatoid arthritis, comprising the steps of:
   a) incubating synoviolin or a homolog thereof and a substrate in the presence of a test compound under enabling conditions for substrate ubiquitination,
   a') contacting either (i) a substrate or (ii) synoviolin or a homolog thereof with a test compound and incubating with the other of (i) and (ii) under enabling conditions for substrate ubiquitination, or
   a") incubating a substrate and synoviolin or a homolog thereof under enabling conditions for substrate ubiquitination and contacting them with a test compound;
   b) measuring level of substrate ubiquitination after any one of steps a), a'), and a"); and
   c) selecting a test compound with a substrate ubiquitination level lower than a ubiquitination level measured in the absence of the test compound as a compound for treatment or prevention of rheumatoid arthritis,
   wherein the substrate is prolyl 4-hydroxylase α subunit or a homolog thereof.
[34] The method of [33], wherein the enabling conditions for substrate ubiquitination are provided by coexistence of the following components:
   i) a ubiquitin activating enzyme;
   ii) a ubiquitin transferase;
   iii) ubiquitin; and
   iv) adenosine triphosphate.
[35] The method of [33], wherein the enabling conditions for substrate ubiquitination are provided by ubiquitinating the substrate in a cell expressing the substrate and the synoviolin or a homolog thereof.
[36] The method of [33], wherein the level of substrate ubiquitination is measured using any one of the following levels as an index:
   A) level of ubiquitinated substrate;
   B) level of unubiquitinated substrate; and
   C) level of prolyl 4-hydroxylase α subunit bioactivity.
[37] The method of [33], wherein the prolyl 4-hydroxylase α subunit or a homolog thereof is any one of the following polypeptides (a) to (e):
   (a) a polypeptide encoded by the nucleotide sequence of SEQ ID NO: 1;
   (b) a polypeptide comprising the amino acid sequence of SEQ ID NO: 2;
   (c) a polypeptide which comprises an amino acid sequence with a substitution, deletion, insertion, and/or addition of one or plural amino acids in the amino acid sequence of SEQ ID NO: 2 and can be ubiquitinated by synoviolin;
   (d) a polypeptide which comprises an amino acid sequence encoded by a polynucleotide hybridizing under stringent conditions with a DNA comprising the nucleotide sequence of SEQ ID NO: 1 and can be ubiquitinated by synoviolin; and
   (e) a polypeptide which comprises an amino acid sequence having a homology of 70% or more with the amino acid sequence of SEQ ID NO: 2 and can be ubiquitinated by synoviolin.
[38] The method of [33], wherein the synoviolin or a homolog thereof is any one of the following polypeptides (A) to (E):
   (A) a polypeptide encoded by the nucleotide sequence of SEQ ID NO: 3;
   (B) a polypeptide comprising the amino acid sequence of SEQ ID NO: 4;
   (C) a polypeptide which comprises an amino acid sequence with a substitution, deletion, insertion, and/or addition of one or plural amino acids in the amino acid sequence of SEQ ID NO: 4 and has an activity of ubiquitinating a polypeptide comprising the amino acid sequence of SEQ ID NO: 2;
   (D) a polypeptide which hybridizes under stringent conditions with a DNA comprising the nucleotide sequence of SEQ ID NO: 3 and that has an activity of ubiquitinating a polypeptide comprising the amino acid sequence of SEQ ID NO: 2; and
   (E) a polypeptide which comprises an amino acid sequence having a homology of 70% or more with the amino acid sequence of SEQ ID NO: 4 and has an activity of ubiquitinating a polypeptide comprising the amino acid sequence of SEQ ID NO: 2.
[39] A pharmaceutical composition for treatment and/or prevention of rheumatoid arthritis, comprising a compound selectable by the method of any one of [33] to [38] as an active ingredient.
[40] A kit for screening for a compound for treatment or prevention of rheumatoid arthritis, comprising the following components:
   (1) synoviolin or a homolog thereof; and
   (2) prolyl 4-hydroxylase α subunit or a homolog thereof.
[41] The kit of [39], further comprising the following components:
   (3) a ubiquitin activating enzyme;
   (4) a ubiquitin transferase;
   (5) ubiquitin; and
   (6) adenosine triphosphate.
[42] A kit for screening for a test compound for treatment or prevention of rheumatoid arthritis, comprising a cell expressing synoviolin or a homolog thereof, and prolyl 4-hydroxylase α subunit or a homolog thereof; and a means for measuring the ubiquitination level of the prolyl 4-hydroxylase α subunit or a homolog thereof.
[43] A method of screening for a compound for treatment or prevention of rheumatoid arthritis, comprising the steps of:
   a) incubating in the presence of a test compound under enabling conditions for binding of the synoviolin or a homolog thereof and the substrate;
   a') contacting either (i) a substrate or (ii) synoviolin or a homolog thereof with a test compound and incubating under enabling conditions for binding of the synoviolin and the substrate, or
   a") incubating under enabling conditions for binding of the substrate and the synoviolin or a homolog thereof and contacting them with a test compound;
   b) measuring the level of binding between the substrate and the synoviolin or a homolog thereof after any one of steps a), a'), and a"); and
   c) selecting a test compound with a binding level between the substrate and the synoviolin or a homolog thereof lower than a binding level measured in the absence of the test compound as a compound for treatment or prevention of rheumatoid arthritis,
   wherein the substrate is prolyl 4-hydroxylase α subunit or a homolog thereof.
[44] The method of [43], wherein either the synoviolin or the substrate is bound to a solid phase or comprises a label capable of binding to a solid phase.
[45] The method of [43], wherein the prolyl 4-hydroxylase α subunit or a homolog thereof is any one of the following polypeptides (a) to (e):
   (a) a polypeptide encoded by the nucleotide sequence of SEQ ID NO: 1;
   (b) a polypeptide comprising the amino acid sequence of SEQ ID NO: 2;
   (c) a polypeptide which comprises an amino acid sequence with a substitution, deletion, insertion, and/or addition of one or plural amino acids in the amino acid sequence of SEQ ID NO: 2 and can bind to synoviolin;
   (d) a polypeptide which comprises an amino acid sequence encoded by a polynucleotide hybridizing under stringent conditions with a DNA comprising the nucleotide sequence of SEQ ID NO: 1 and can bind to synoviolin; and
   (e) a polypeptide which comprises an amino acid sequence having a homology of 70% or more with the amino acid sequence of SEQ ID NO: 2 and can bind to synoviolin.
[46] The method of [43], wherein the synoviolin or a homolog thereof is any one of the following polypeptides (A) to (E):
   (A) a polypeptide encoded by the nucleotide sequence of SEQ ID NO: 3;
   (B) a polypeptide comprising the amino acid sequence of SEQ ID NO: 4;
   (C) a polypeptide which comprises an amino acid sequence with a substitution, deletion, insertion, and/or addition of one or plural amino acids in the amino acid sequence of SEQ ID NO: 4 and can bind to a polypeptide comprising the amino acid sequence of SEQ ID NO: 2;
   (D) a polypeptide which hybridizes under stringent conditions with a DNA comprising the nucleotide sequence of SEQ ID NO: 3 and that can bind to a polypeptide comprising the amino acid sequence of SEQ ID NO: 2; and
   (E) a polypeptide which comprises an amino acid sequence having a homology of 70% or more with the amino acid sequence of SEQ ID NO: 4 and can bind to a polypeptide comprising the amino acid sequence of SEQ ID NO: 2.
[47] A pharmaceutical composition for treatment and/or prevention of rheumatoid arthritis, comprising a compound selectable by the method of any one of [43] to [46] as an active ingredient.
   Furthermore, the present inventors conducted a screen of test compounds using the methods of the present invention and isolated the polypeptide of SEQ ID NO: 5. Specifically, the present invention relates to the following polypeptides, polynucleotides encoding the polypeptides, and pharmaceutical compositions comprising the polypeptides as an active ingredient.
[48] A polypeptide comprising the amino acid sequence of SEQ ID NO: 5.
[49] A polypeptide comprising an amino acid sequence with a substitution, deletion, insertion, and/or addition of one or plural amino acids in the amino acid sequence of SEQ ID NO: 5,
   wherein when the polypeptide is used as a test compound in the method of [1], the ubiquitination level of a substrate is lower than a ubiquitination level binding level measured in the absence of the polypeptide.
[50] A polypeptide comprising an amino acid sequence with a substitution, deletion, insertion, and/or addition of one or plural amino acids in the amino acid sequence of SEQ ID NO: 5,
   wherein when the polypeptide is used as a test compound in the method of [12], the level of binding between a substrate and synoviolin or a homolog thereof is lower than a binding level measured in the absence of the polypeptide.
[51] A polynucleotide encoding the polypeptide of any one of [48] to [50].
[52] A pharmaceutical composition for treatment and/or prevention of fibrosis, comprising the polypeptide of any one of claims 48 to 50 as an active ingredient.
[53] A pharmaceutical composition for treatment and/or prevention of rheumatoid arthritis, comprising the polypeptide of any one of claims 48 to 50 as an active ingredient.

The present invention is based on the novel fmding that prolyl 4-hydroxylase activity is controlled by the synoviolin ubiquitination of proline hydroxylase α subunit. Namely, the present invention provides methods for detecting the regulatory activity of a test compound on the ubiquitination effect of synoviolin, comprising the steps of:
a) incubating a substrate and synoviolin or a homolog thereof in the presence of a test compound under enabling conditions for substrate ubiquitination,
a') contacting either (i) a substrate or (ii) synoviolin or a homolog thereof with a test compound and incubating with the other of (i) and (ii) under enabling conditions for substrate ubiquitination, or
a") incubating a substrate and synoviolin or a homolog thereof under enabling conditions for substrate ubiquitination and contacting them with a test compound;
b) measuring level of substrate ubiquitination after any one of steps a), a'), and a"); and
c) detecting regulatory activity of the test compound on the synoviolin ubiquitination of the substrate when the substrate ubiquitination level differs from a ubiquitination level measured in the absence of the test compound,
wherein the substrate is P4HA1 or a homolog thereof.

"Ubiquitination" refers to the binding of at least one ubiquitin. "Ubiquitination level" refers to the number of conjugated ubiquitins, or the number of sites to which ubiquitin is conjugated, or both. Namely, the ubiquitination level can be determined by measuring the number of ubiquitins conjugated to a specific portion of a certain polypeptide. Furthermore, when multiple regions of a certain polypeptide are ubiquitinated, the ubiquitination level can also be determined by measuring the number of ubiquitinated regions.

Methods for determining the ubiquitination level are known. For example, the ubiquitination level can be determined by using substances that have an affinity for and bind to ubiquitin. An anti-ubiquitin antibody can be used as a substance having such affinity. For example, monoclonal antibodies that specifically recognize and bind ubiquitin are commercially available. By labeling a substance having affinity for ubiquitin, the ubiquitination level of a substrate can be determined based on the level of the labeling substance bound to the substrate. Alternatively, by supplying a prelabeled ubiquitin, the amount of ubiquitin bound to a substrate polypeptide can be measured based on the level of the labeling substance.

Commonly used labeling techniques can be adopted for the labeling of such antibodies and ubiquitin. Specifically, radioactive substances, enzymatically active substances, fluorescent active substances, and light-emitting substances, for example, can be used as a label. Binding tags can be used to indirectly bind a label with an antibody or ubiquitin. For example, an avidinylated label can be bound to a biotinylated antibody or ubiquitin. Furthermore, a ubiquitinated substrate can also be captured by using ubiquitin introduced with a binding tag. By measuring the level of the captured substrate polypeptide, the ubiquitination level can be evaluated. In this method, the ubiquitination level is measured by using a labeled antibody against the substrate polypeptide. The following labeled ubiquitins are commercially available for measurement of the ubiquitination level.

Fluorescein-binding ubiquitin: An ubiquitin bound to fluorescein (i.e., a fluorescent substance). The ubiquitination level can be measured using fluorescence intensity as an index.

Biotinylated ubiquitin: A ubiquitin labeled with biotin (i.e., a binding ligand). A substrate polypeptide ubiquitinated by biotinylated ubiquitin can be captured with an avidin column. Alternatively, the ubiquitination level can be measured by using an avidinylated enzyme, a fluorescent substance or such.

(His) 6-ubiquitin: A ubiquitin labeled with a histidine tag. The histidine tag can be captured with a nickel column. Namely, a ubiquitinated substrate can be captured on a column via ubiquitin. Alternatively, indirect labeling is possible by using an anti-histidine tag antibody.

It is advantageous to solid-phase the substrate when measuring ubiquitination levels. For example, the substrate can be bound to a solid phase in advance. Specifically, the substrate can be chemically bound to, or physically adsorbed to beads or the inner wall of a reactor. For the beads or reactor, for example, polystyrene can be used. Polystyrene derivatives that have been introduced with functional groups, such as amino groups and carboxyl groups, to be used for the chemical binding of substrates are also known.

Alternatively, a ubiquitinated substrate can be indirectly captured via ubiquitin by using the above-mentioned ubiquitin labeled with a substance having binding affinity. Vice versa, a substrate introduced with an antibody against the substrate or a substance with binding affinity for the substrate can be captured on a solid phase. In either case, the solid-phased substrate is separated from unbound components, and the ubiquitination level is measured. When ubiquitin or the substrate itself is not labeled, a label can be linked thereto by using a substance having affinity for ubiquitin or the substrate. For example, antibodies against ubiquitin are used. Finally, the ubiquitination level can be determined by measuring the level of the label captured by the solid phase (or unbound label). From the measurements of the ubiquitination level, the amount of ubiquitinated substrate can be determined. Alternatively, the amount of unubiquitinated substrate can also be determined by measuring the ubiquitination level.

In addition, the present invention has also revealed that the bioactivity of prolyl 4-hydroxylase is regulated by the ubiquitination of P4HA1. Consequently, the ubiquitination level can also be measured by using the bioactivity level of prolyl 4-hydroxylase as an index. The bioactivity level of prolyl 4-hydroxylase can be determined, for example, by a tritium release assay (Methods Enzymol., Vol. 82, pp. 246-259, 1982).

The order of contacting synoviolin (or a homolog thereof), a substrate, and a test substance is arbitrary. Namely, the present invention can comprise the step of contacting synoviolin (or a homolog thereof), a substrate, and a test substance in any order of the steps a), a'), and a") mentioned above. When the three components are incubated in the presence of test substance (a), effects of the test substance on ubiquitination reactions by synoviolin (or a homolog thereof) can be evaluated. Alternatively, the test substance can be conditioned to enable ubiquitination after being contacted with either one of synoviolin (or a homolog thereof) and substrate (a'). In this case, effects of the test substance on the ubiquitin ligase activity of synoviolin or the substrate function of being ubiquitinated are evaluated. Furthermore, by contacting the test substance after ubiquitination-enabling conditions are applied (a"), effects of the test substance on the ubiquitinated substrate can be evaluated.

P4HA1 or a homolog thereof is used as a substrate in the present invention. P4HA1 is one of the subunits that constitute the tetramer prolyl 4-hydroxylase. The α subunit is considered to have a function of catalyzing the hydroxylation reaction of proline. The homologs thereof are polypeptides that have a structural similarity to P4HA1 and can be ubiquitinated by synoviolin. For P4HA1 of the present invention or homologs thereof, for example, the following polypeptides (a) to (e) can be used:
(a) a polypeptide encoded by the nucleotide sequence of SEQ ID NO: 1;
(b) a polypeptide comprising the amino acid sequence of SEQ ID NO: 2;
(c) a polypeptide that comprises an amino acid sequence with a substitution, deletion, insertion, and/or addition of one or plural amino acids in the amino acid sequence of SEQ ID NO: 2 and which can be ubiquitinated by synoviolin;
(d) a polypeptide that comprises an amino acid sequence encoded by a polynucleotide hybridizing under stringent conditions with a DNA comprising the nucleotide sequence of SEQ ID NO: 1 and which can be ubiquitinated by synoviolin; and
(e) a polypeptide that comprises an amino acid sequence having a homology of 70% or more with the amino acid sequence of SEQ ID NO: 2 and which can be ubiquitinated by synoviolin.

Polynucleotides comprising a nucleotide sequence with mutations in the polynucleotide comprising the nucleotide sequence of SEQ ID NO: 1 can be isolated by one skilled in the art according to known methods (Jikken Igaku (Experimental Medicine) Supplementary Volume, Genetic Engineering Handbook, pp 246-251, Yodosha Co., Ltd., 1991). For example, a DNA comprising a highly homologous nucleotide sequence can be cloned by screening a library comprising analogous genes using the nucleotide sequence of SEQ ID NO: 1 (or a fragment thereof) as a probe. A library of genes with randomly added mutations in the nucleotide sequence of SEQ ID NO: 1 and a cDNA library derived from a nonhuman species are examples of such a library.

For example, substitution of base pairs by treating DNA with nitrous acid is known as a method for randomly adding mutations to a given nucleotide sequence (Proc. Natl. Acad. Sci. USA., 79:7258-7260, 1982). According to this method, a base pair substitution can be randomly introduced into a segment by treating the segment with nitrous acid. Alternatively, as a technique for introducing a target mutation into an arbitrary site (Methods Enzymol., 154:350-367, 1987), the gapped duplex method and such are known. A cyclic double-stranded vector, which has been prepared by cloning a gene to which a mutation is to be introduced, is converted into a single strand, and a synthetic oligonucleotide comprising a mutation at the target site is hybridized. Complementary single-stranded DNAs derived from the vector are linearized by restriction enzyme cleavage and annealed to the cyclic single-stranded vector, wherein gaps between the vector and the synthetic nucleotides are filled using DNA polymerase, and ligation is conducted. Thus, a complete double-stranded cyclic vector is obtained.

The number of amino acids to be modified is typically 50 amino acids or less, preferably 30 amino acids or less, more preferably 5 amino acids or less (for example, one amino acid), or several amino acids. When artificially substituting an amino acid, it may be easier to maintain the activity of the original protein by substituting the amino acid with another amino acid that has similar characteristics. The proteins of the present invention include polypeptides that comprise conservative substitutions in addition to the above amino acid substitutions and which are functionally equivalent to P4HA1 (SEQ ID NO: 2). Conservative substitution is considered important, for example, when substituting an amino acid in a domain that is essential for protein activity. Conservative substitution of such an amino acid is well known by one skilled in the art.

Examples of amino acid groups appropriate for conservative substitution are basic amino acids such as lysine, arginine, and histidine; acidic amino acids such as aspartic acid and glutamic acid; uncharged polar amino acids such as glycine, asparagine, glutamine, serine, threonine, tyrosine, and cysteine; non-polar amino acids such as alanine, valine, leucine, isoleucine, proline, phenylalanine, methionine, and tryptophan; β-branched amino acids such as threonine, valine, and isoleucine; and aromatic amino acids such as tyrosine, phenylalanine, tryptophan, and histidine.

The activity of a protein can be further increased (e.g., a constantly active protein) or decreased (e.g., a dominant negative) by non-conservative substitution.

Polypeptides which comprise an amino acid sequence with a substitution, deletion, insertion, and/or addition of one or plural amino acids in the amino acid sequence of SEQ ID NO: 2 and are functionally equivalent to a polypeptide comprising the amino acid sequence of SEQ ID NO: 2 include artificially synthesized and naturally-occurring polypeptides. The "plural amino acids" herein refers to typically 50 amino acids or less, preferably 30 amino acids or less, more preferably 5 amino acids or less (for example, 1 amino acid) and several amino acids. Eukaryotic genes generally have polymorphism as in interferon genes. Nucleotide sequence changes resulted from polymorphism may lead to substitution, deletion, insertion, and/or addition of one or plural amino acids. Naturally-occurring polypeptides comprising an amino acid sequence with a substitution, deletion, insertion, and/or addition of one or plural amino acids in the amino acid sequence of SEQ ID NO: 2 and which are functionally equivalent to a polypeptide comprising the amino acid sequence of SEQ ID NO: 2 can be used in the present invention.

Even if a nucleotide sequence changes as a result of polymorphism, there may not be any changes in the amino acid sequence. Nucleotide sequence mutations of this type are called silent mutations. Genes consisted of a nucleotide sequence with a silent mutation can also be used in the present invention. The "polymorphism" used herein means that a certain gene has nucleotide sequences that differ in a group from individual to individual. Polymorphism is not related to the ratio of different genes being discovered.

In addition, a method that uses hybridization can be used as the method for obtaining P4HA1 protein or a homolog thereof. Specifically, a P4HA1-encoding polynucleotide as represented by SEQ ID NO: 1, or a fragment thereof, can be used as a probe to isolate polynucleotides that are capable of hybridizing with the probe. By conducting the hybridization under stringent conditions, polynucleotides with high nucleotide sequence homology can be selected. The resulting isolated proteins are more likely to include the P4HA1 protein or a homolog thereof. The "high nucleotide sequence homology" refers to, for example, an identity of 70% or more and preferably 90% or more.

The stringent conditions include, specifically, for example, conditions of 6x SSC, 40% formamide, hybridization at 25°C and washing with 1x SSC, at 55°C. The stringency varies depending on conditions such as salt concentration, formamide concentration, and temperature. It is obvious for one skilled in the art to adjust these conditions so as to yield required stringency.

By using hybridization, for example, polynucleotides encoding a nonhuman homolog of P4HA1 can be isolated. P4HA1 homologs encoded by polynucleotides derived from nonhuman animals, such as mice, rats, rabbits, pigs, or goats, constitute functionally equivalent proteins in the present invention.

Polynucleotides of the present invention can have any origin. Namely, they can be obtained from cDNAs, genomic DNAs, or synthetically prepared. Additionally, polynucleotides having an arbitrary nucleotide sequence based on degeneracy of the genetic code are also included, as long as they can encode proteins of the present invention.

Proteins obtained by introducing mutations into human P4HA1 (SEQ ID NO: 2) and proteins encoded by polynucleotides isolated by the above hybridization techniques generally have a high amino acid sequence homology with human P4HA1 (SEQ ID NO: 2). The "high homology" refers to a sequence identity of 30% or more, preferably 50% or more, and more preferably 80% or more (for example, 95% or more). The nucleotide sequence or amino acid sequence identity can be determined by using an internet homology search site [for example, homology searches such as FASTA, BLAST, PSI-BLAST, and SSEARCH in DNA Data Bank of Japan (DDBJ) can be used (for example, a homology search (Search and Analysis) page on the website of DNA Data Bank of Japan (DDBJ); http://www.ddbj.nig.ac.jp/E-mail/homology-j.html). In addition, a search using BLAST can be carried out at the National Center for Biotechnology Information (NCBI) (for example, a BLAST page on the website of NCBI; http://www.ncbi.nlm.nih.govBLAST/; Altschul, S.F. *et al.,* J. Mol. Biol., 1990, 215(3):403-10; Altschul, S.F. & Gish, W., Meth. Enzymol., 1996, 266:460-480; Altschul, S.F. *et al.,* Nucleic Acids Res., 1997, 25:3389-3402)].

Synoviolin or a homolog thereof is used as a ubiquitin ligase (E3) in the present invention. Synoviolin is a polypeptide comprising a RING fmger motif. The amino acid sequence thereof (SEQ ID NO: 4), and the nucleotide sequence (SEQ ID NO: 3) encoding the amino acid sequence were identified. Meanwhile, the "homolog thereof' is a polypeptide that has structural resemblance with synoviolin and can ubiquitinate P4HA1. For example, the following polypeptides (A) to (E) can be used as "synoviolin or a homolog thereof' in the present invention:
(A) a polypeptide encoded by the nucleotide sequence of SEQ ID NO: 3;
(B) a polypeptide comprising the amino acid sequence of SEQ ID NO: 4;
(C) a polypeptide that comprises an amino acid sequence with a substitution, deletion, insertion, and/or addition of one or plural amino acids in the amino acid sequence of SEQ ID NO: 4 and which has an activity of ubiquitinating a polypeptide comprising the amino acid sequence of SEQ ID NO: 2;
(D) a polypeptide which hybridizes under stringent conditions with a DNA comprising the nucleotide sequence of SEQ ID NO: 3 and that has an activity of ubiquitinating a polypeptide comprising the amino acid sequence of SEQ ID NO: 2; and
(E) a polypeptide which comprises an amino acid sequence having a homology of 70% or more with the amino acid sequence of SEQ ID NO: 4 and has an activity of ubiquitinating a polypeptide comprising the amino acid sequence of SEQ ID NO: 2.

Methods for obtaining functionally equivalent polypeptides based on information of known amino acid sequences and information of known nucleotide sequences are described above. The desirable structural conditions for the homologs are as mentioned above. The thus obtainable homologs can be used as synoviolin in the present invention. For example, WO 02/052007 discloses various synoviolin homologs. These homologs can be used in the methods of the present invention, as long as they have a function of ubiquitinating P4HA1.

Polypeptides that comprise an amino acid sequence with a substitution, deletion, insertion, and/or addition of one or plural amino acids in the amino acid sequence of SEQ ID NO: 4 and which are functionally equivalent to a polypeptide comprising the amino acid sequence of SEQ ID NO: 2 include artificially synthesized and naturally-occurring polypeptides. The "plural amino acids" herein refers to typically 50 amino acids or less, preferably 30 amino acids or less, more preferably 5 amino acids or less (for example, 1 amino acid) and several amino acids. Eukaryotic genes generally comprise polymorphism as in interferon genes. Nucleotide sequence changes resulted from polymorphism may lead to the substitution, deletion, insertion, and/or addition of one or plural amino acids. Naturally-occurring proteins that comprise an amino acid sequence with a substitution, deletion, insertion, and/or addition of one or plural amino acids in the amino acid sequence of SEQ ID NO: 4 and which are functionally equivalent to a protein comprising the amino acid sequence of SEQ ID NO: 4 can be used in the present invention.

Even if a nucleotide sequence changes as a result of polymorphism, there may not be any changes in the amino acid sequence. Nucleotide sequence mutations of this type are called silent mutations. Genes consisted of a nucleotide sequence with a silent mutation are also included in the present invention. The "polymorphism" used herein means that a certain gene has nucleotide sequences that differ in a group from individual to individual. Polymorphism is not related to the ratio of different genes being discovered.

In addition, methods that utilize hybridization can be used as a method for obtaining synoviolin protein or a homolog thereof. Specifically, a synoviolin-encoding polynucleotide as represented by SEQ ID NO: 3 in the present invention or a fragment thereof can be used as a probe to isolate polynucleotides that are capable of hybridizing with the probe. By conducting the hybridization under stringent conditions, polynucleotides with high nucleotide sequence homology can be selected. The resulting isolated proteins are more likely to include synoviolin protein or a homolog thereof. The "high nucleotide sequence homology" refers to, for example, an identity of 70% or more and preferably 90% or more.

The stringent conditions include, specifically, for example, conditions of 6x SSC, 40% formamide, hybridization at 25°C and washing with 1x SSC, at 55°C. The stringency varies depending on conditions such as salt concentration, formamide concentration, and temperature. It is obvious for one skilled in the art to adjust these conditions so as to yield required stringency.

By using hybridization, for example, polynucleotides encoding a nonhuman homolog of synoviolin can be isolated. Synoviolin homologs encoded by polynucleotides derived from nonhuman animals, such as mice, rats, rabbits, pigs, or goats, constitute functionally equivalent proteins in the present invention.

Polynucleotides of the present invention can have any origin. Namely, they can be obtained from cDNAs, genomic DNAs, or synthetically prepared. Additionally, polynucleotides having an arbitrary nucleotide sequence based on degeneracy of the genetic code are also included, as long as they can encode proteins of the present invention.

Proteins obtained by introducing a mutation into human synoviolin (SEQ ID NO: 4) and proteins encoded by polynucleotides isolated by hybridization techniques above generally have high amino acid sequence homology with human synoviolin (SEQ ID NO: 4). The "high homology" refers to a sequence identity of 30% or more, preferably 50% or more, and more preferably 80% or more (for example, 95% or more). The nucleotide sequence or amino acid sequence identity can be determined by using an internet homology research site [for example, homology searches such as FASTA, BLAST, PSI-BLAST, and SSEARCH in DNA Data Bank of Japan (DDBJ) can be used (for example, a homology search (Search and Analysis) page on the website of DNA Data Bank of Japan (DDBJ);
http://www.ddbj.nig.ac.jp/E-mail/homology j.html). In addition, a search using BLAST can be carried out at the National Center for Biotechnology Information (NCBI) (for example, a BLAST page on the website of NCBI; http://www.ncbi.nlm.nih.govBLAST/; Altschul, S.F. *et al.,* J. Mol. Biol., 1990, 215(3):403-10; Altschul, S.F. & Gish, W., Meth. Enzymol., 1996, 266:460-480; Altschul, S.F. *et al.,* Nucleic Acids Res., 1997, 25:3389-3402)].

In fact, the present inventors disclosed in WO 02/052007 a clone with one amino acid deletion in a synoviolin-constituting amino acid sequence. Proteins comprising a mutation in the amino acid sequence are included in the synoviolin homologs of the present invention, as long as they have the required functions of the present invention. The mutant which has been confirmed by the present inventors to have one amino acid deletion lacks gca of the corresponding 1293-1295 in SEQ ID NO: 3 and therefore Ala at position 412 in SEQ ID NO: 4.

In the present invention, the P4HA1-ubiquitinating function of a certain protein can be confirmed by contacting the protein with a substrate under ubiquitination-enabling conditions such as those mentioned above and detecting for substrate ubiquitination.

Next, the phrase "enabling conditions for the ubiquitination of a substrate or a homolog thereof' in the present invention refers to conditions under which P4HA1 can be ubiquitinated by synoviolin. Such conditions can be constituted *in vitro* or *in vivo.* For example, enabling conditions for the ubiquitination of a substrate are provided by the coexistence of the following components:
(i) a ubiquitin activating enzyme (E1);
(ii) a ubiquitin transferase (E2);
(iii) ubiquitin; and
(iv) adenosine triphosphate.

The ubiquitination of a protein in the living body is generally considered to proceed in the following manner. The ubiquitination of a protein requires a ubiquitin ligase (ligase/E3), in addition to the four components mentioned above. The ubiquitin activating enzyme (E1), ubiquitin transferase (E2), and ubiquitin ligase (ligase /E3) constitute a multicomponent enzymatic system referred to as a ubiquitin system. Some substrates are ubiquitinated by E2, but P4HA1 is ubiquitinated by synoviolin (E3).

The ubiquitin activating enzyme (E1) activates the ubiquitin transferase (E2). Meanwhile, the ubiquitin ligase (ligase/E3) recognizes and captures a substrate protein, and ubiquitinates the captured substrate protein using ubiquitin of the activated ubiquitin transferase (E2). In the ubiquitin system, the ubiquitin ligase (ligase/E3) has an important role of identifying the substrate protein. Namely, the selection of a substrate by the ubiquitin ligase (ligase/E3) means the end of the substrate protein.

In the ubiquitin system, it is important to combine a substrate with a ubiquitin ligase (ligase/E3) that recognizes the substrate. Consequently, the substrate in the present invention must be P4HA1 or a homolog thereof. At the same time, the ubiquitin ligase (ligase/E3) must be synoviolin or a homolog thereof. The other enzymes, however, can be arbitrary enzymes that enable ubiquitination of the substrate.

For example, a yeast-derived enzyme was used as the ubiquitin activating enzyme (E1) and a human-derived UbcH5c was used as the ubiquitin transferase (E2) in the Examples. The ubiquitin activating enzyme (E1) in the present invention, however, is not limited to such a yeast-derived enzyme. For example, a rat-derived ubiquitin activating enzyme (E1) can be used in the present invention. Likewise, the ubiquitin transferase (E2) is not limited to a human-derived enzyme. E1 and/or E2 can be any naturally-occurring enzymes or recombinants. These recombinants can be modified in structure, as long as they can maintain the activity of E1 or E2. The structural modifications include substitution, deletion, insertion, and addition of amino acid sequences. For example, an E2 prepared by expressing a human-derived UbcH5c as a GST-fused protein in *Escherichia coli* is used in the Examples below. Such recombinants are also included in the E2 of the present invention.

When components (i) to (iv) are used to provide enabling conditions for the ubiquitination of substrates by synoviolin, one skilled in the art can design the other conditions that are required for the ubiquitination. Specifically, one skilled in the art can decide the incubation conditions and the amount of each component to be used. Examples of these conditions are shown by using the components, for example, in the following concentrations:
Synoviolin (E3): 500 ng
GST P4HA1: 800 ng
   (i) Ubiquitin activating enzyme (E1): 60 ng
   (ii) Ubiquitin transferase (E2): 0.3 mg
   (iii) Ubiquitin (³²P-labeled His-ubiquitin): 0.75 µg

Enabling conditions for the ubiquitination of substrates can be established by adding the above-mentioned composition to the following reaction buffer to yield a 30 µL reaction mixture, and incubating this mixture at 37°C for 1 hour. The concentrations of the components can be appropriately adjusted when necessary. For example, the concentrations can be adjusted within the range of one-tenths to ten times the above-mentioned amounts.

### Composition of Reaction Buffer

50 mM Tris-HCl buffer pH 7.4
5 mM MgCl₂
2 mM NaF
10 nM Okadaic acid
2 mM ATP (iv)
0.6 mM Dithiothreitol (DTT)

Alternatively, ubiquitination-enabling conditions of the present invention can be *constituted in vivo.* Namely, enabling conditions for the ubiquitination of a substrate can be provided by ubiquitinating the substrate in a cell expressing the substrate and synoviolin or a homolog thereof. For example, synovial cells can be used as a cell to express synoviolin and the substrate.

Alternatively, ubiquitination-enabling conditions of the present invention can be provided by cells in which synoviolin (or a homolog thereof) and a substrate are forcedly expressed through genetic engineering. In cells introduced with a gene encoding synoviolin (or a homolog thereof) or a substrate as an exogenous gene, it is possible to control the expression timing of synoviolin or the substrate by using an inducible expression system. Living cells have ubiquitin systems. That is, components (i) to (iv) are present in the cell. Consequently, by allowing synoviolin and the substrate to exist, ubiquitination-enabling conditions of the present invention can be constituted.

The nucleotide sequences encoding synoviolin and P4HA1 (a substrate thereof) are known. One skilled in the art can design the expression system based on known genetic information. Animal cells, insect cells, yeasts, or such can be used as the host cell for forced expression.

A ubiquitinated substrate in a cell can be recovered by destructing the cell. Furthermore, the target substrate can be isolated by using a substance having affinity for ubiquitin or the substrate. Many ubiquitinated proteins besides the target substrate are present in the cell. Consequently, when a substrate is ubiquitinated in a cell, it is preferred that the ubiquitination of the target substrate protein is specifically determined.

For example, a method that uses an antibody against a substrate protein to specifically capture the substrate protein can be used for that purpose. Alternatively, it is possible to isolate a substrate protein by expressing it as a fused protein with a tag peptide, such as histidine tag, and using the tag as a marker. The substrate protein thus recovered from the cell is then determined for its ubiquitination level by the methods mentioned above.

For example, ubiquitination-enabling conditions can be constituted by co-transforming cells that express P4HA1 (or a homolog thereof) with tagged ubiquitin and synoviolin (or a homolog thereof). The ubiquitination level can be determined by carrying out immunoprecipitation using an antibody against the tag and measuring the P4HA1 level in the precipitated fraction. The P4HA1 level in the precipitated fraction can be immunologically measured using an antibody against P4HA1. Alternatively, an unubiquitinated substrate can be determined by measuring the level of unprecipitated P4HA1 following immunoprecipitation.

Furthermore, ubiquitination-enabling conditions can be constituted by co-transformaing P4HA1-expressing cells with tagged ubiquitin and synoviolin (or a homolog thereof). After immunoprecipitation with an anti-P4HA1 antibody, the ubiquitination level can be determined by using an antibody against the tag or an antibody against ubiquitin. For example, synovial cells can be used as the P4HA1-expressing cell.

The present invention also relates to methods of screening for a test compound having regulatory activity on the ubiquitination effect of synoviolin, comprising the following steps:
a) using the methods of the present invention for detecting regulatory activity on the ubiquitination effect of synoviolin to detect regulatory activity of a test compound on the ubiquitination effect of synoviolin; and
b) selecting a test compound with a differential ubiquitination level of the substrate as compared with a control.

A more specific example of the screening methods according to the present invention is illustrated below.

Most commonly, a substance affecting the interaction between synoviolin and P4HA1 can be selected by reacting synoviolin (an enzyme) with P4HA1 (a substrate thereof) in the presence of a test compound and measuring the amount reacted. In this case, a more objective selection is possible by comparing the result with a control in which synoviolin and P4HA1 are reacted in the absence of the test compound.

Synoviolin catalyzes the ubiquitination reaction of P4HA1. Accordingly, the amount reacted can be determined by conducting the reaction in the presence of ubiquitin and measuring the amount of ubiquitinated P4HA1. The amount of ubiquitinated P4HA1, namely the amount of ubiquitin bound to P4HA1 can be measured, for example, by methods using a substance that specifically binds to ubiquitin, such as an antibody against ubiquitin. An anti-ubiquitin antibody can be obtained according to conventional procedures. For example, ubiquitin is injected to an immune animal as an immunogen, and the serum is collected from the blood of the animal. Mice, rats, rabbits, or such can be used as the immune animal. Monoclonal antibodies can be used as the antibody. Methods for obtaining such monoclonal antibodies are known.

Moreover, ubiquitin can be measured by labeling ubiquitin itself and detecting or measuring the labeled ubiquitin. For the label used herein, general labels can be used. Specifically, examples of the label include a radioactive label, an enzymatic label, biotin and such. When ubiquitin is labeled with ³²P, for example, it can be detected by autoradiography. The radioactivity of ³²P can also be measured with a scintillation counter. Biotin-labeled ubiquitin can be detected using a labeled avidin.

The specific methods can be illustrated by the following methods.

### Method 1.

A tagged P4HA1 is prepared, and this is incubated with E1, E2, ATP, synoviolin, ubiquitin and a test compound to form a P4HA1-ubiquitin complex. Next, an antibody against the tag of P4HA1 is reacted, and the complex is separated from unreacted ubiquitin by immunoprecipitation. Furthermore, the ubiquitin that forms the complex is detected or measured using an anti-ubiquitin antibody. A reaction system that does not comprise the test compound is detected or measured in the same way as above, and the amounts of bound ubiquitin are compared between the presence and absence of the test compound.

### Method 2.

P4HA1, E1, E2, ATP, synoviolin, [³²P]-labeled ubiquitin, and a test compound are incubated together to form a P4HA1-ubiquitin complex. Next, the complex and unreacted ubiquitin are separated from each other by electrophoresis and such. Furthermore, the complex-forming ubiquitin is detected or measured by autoradiography. A reaction system that does not comprise the test compound is detected or measured in the same way as above, and the amounts of bound ubiquitin are compared between the presence and absence of the test compound.

In the above-mentioned method, biotin-labeled ubiquitin can be used instead of ³²P-labeled ubiquitin. In this case, ubiquitin is detected or measured using a labeled avidin. Horseradish peroxidase, alkaline phosphatase, fluorescence, or such can be used as the label. Method 3.

Synoviolin, E1, E2, ATP, P4HA1 linked to liquid scintillant-containing beads, [³³P]- or [³²P]-labeled ubiquitin, and a test compound are reacted, and the exciting light emitted upon binding of the labeled ubiquitin with P4HA1 is detected or measured using a liquid scintillation counter. A reaction system that does not comprise the test compound is detected or measured in the same way as above, and the amounts of ubiquitin bound are compared between the presence and absence of the test compound.

In Methods 1 to 3, when the test compound is a substance (antagonist) that inhibits the activity of synoviolin, i.e., the interaction between synoviolin and P4HA1, the amount of ubiquitin bound in the presence of the test compound is less than that in the absence of the test compound. When the test compound is a substance (agonist) that activates the interaction between synoviolin and P4HA1, the result is opposite. Consequently, the results of the above experiments can be used to determine whether the test compound is an agonist (activator) or an antagonist (inhibitor).

The present inventors revealed that synoviolin binds to and ubiquitinates P4HA1. Consequently, regulation of the synoviolin ubiquitination of P4HA1 can be evaluated by detecting interference of the binding between the two. Namely, the present invention relates to methods for detecting the regulatory activity of a test compound on the ubiquitination effect of synoviolin, comprising the steps of:
a) incubating in the presence of a test compound under enabling conditions for binding of the synoviolin or a homolog thereof and the substrate,
a') contacting either (i) a substrate or (ii) synoviolin or a homolog thereof with a test compound and incubating under enabling conditions for binding of the synoviolin and the substrate, or
a") incubating under enabling conditions for binding of the substrate and the synoviolin or a homolog thereof and contacting them with a test compound;
b) measuring the level of binding between the substrate and the synoviolin or a homolog thereof after any one of steps a), a'), and a"); and
c) detecting regulatory activity of the test compound on the synoviolin ubiquitination of the substrate when the level of binding between the synoviolin or a homolog thereof and the substrate differs from a binding level measured in the absence of the test compound,
wherein the substrate is prolyl 4-hydroxylase α subunit or a homolog thereof.

The order of contacting synoviolin (or a homolog thereof), a substrate, and a test substance is arbitrary. Namely, the present invention can comprise the step of contacting synoviolin (or a homolog thereof), a substrate, and a test substance in any order described in steps a), a'), and a") mentioned above. When the three components are incubated in the presence of a test substance (a), the effect of the test substance on the binding between synoviolin (or homologs thereof) and the substrate can be evaluated. Alternatively, enabling conditions for the binding between synoviolin (or a homolog thereof) and a substrate can be provided after contacting the test substance with synoviolin (or homologs thereof) or substrate (a'). In this case, the effect of the test substance on the binding ability of synoviolin or that of the substrate is evaluated. Furthermore, by contacting the test substance after enabling conditions for the binding between synoviolin and the substrate are provided (a"), the effect of the test substance on the complex of synoviolin and the substrate can be evaluated.

Either the (i) substrate or (ii) synoviolin or a homolog thereof is preferably linked to a solid phase or preferably comprises a label capable of binding to a solid phase. A label capable of binding to a solid phase can be, for example, a binding ligand. Binding ligands include biotin and such. Biotin can be captured on a solid phase such as avidin-Sepharose. Binding products of the two are easily separated by binding to a solid phase.

The other components which have not been solid-phased can be labeled for easy detection. Synoviolin, homologs thereof, or the substrates can be labeled according to conventional procedures for protein labeling. Specifically, radioactive substances, enzymatically active substances, fluorescent active substances, light-emitting substances, and such can be used as a label. Binding tags can be used to allow indirect binding of a label to a protein. For example, an avidinylated label can be bound to a biotinylated protein.

In the methods of the present invention, any arbitrary protein having a binding activity with synoviolin can be used as P4HA1 or a homolog thereof. Specifically, P4HA1 or homologs thereof comprise any of the following polypeptides (a) to (e):
(a) a polypeptide encoded by the nucleotide sequence of SEQ ID NO: 1;
(b) a polypeptide comprising the amino acid sequence of SEQ ID NO: 2;
(c) a polypeptide which comprises an amino acid sequence with a substitution, deletion, insertion, and/or addition of one or plural amino acids in the amino acid sequence of SEQ ID NO: 2 and can bind to synoviolin;
(d) a polypeptide which comprises an amino acid sequence encoded by a polynucleotide hybridizing under stringent conditions with a DNA comprising the nucleotide sequence of SEQ ID NO: 1 and can bind to synoviolin; and
(e) a polypeptide which comprises an amino acid sequence having a homology of 70% or more with the amino acid sequence of SEQ ID NO: 2 and can bind to synoviolin.

Any arbitrary protein having a binding activity with P4HA1 can be used as synoviolin or a homolog thereof in the methods of the present invention. Specifically, synoviolin or homologs thereof comprise any one of the following polypeptides (A) to (E):
(A) a polypeptide encoded by the nucleotide sequence of SEQ ID NO: 3;
(B) a polypeptide comprising the amino acid sequence of SEQ ID NO: 4;
(C) a polypeptide which comprises an amino acid sequence with a substitution, deletion, insertion, and/or addition of one or plural amino acids in the amino acid sequence of SEQ ID NO: 4 and can bind to a polypeptide comprising the amino acid sequence of SEQ ID NO: 2;
(D) a polypeptide which hybridizes under stringent conditions with a DNA comprising the nucleotide sequence of SEQ ID NO: 3 and that can bind to a polypeptide comprising the amino acid sequence of SEQ ID NO: 2; and
(E) a polypeptide which comprises an amino acid sequence having a homology of 70% or more with the amino acid sequence of SEQ ID NO: 4 and can bind to a polypeptide comprising the amino acid sequence of SEQ ID NO: 2.

Methods for introducing an arbitrary mutation into a given amino acid sequence are as described above. Methods that use hybridization or PCR for isolating a polynucleotide which encodes a structurally analogous polypeptide from polynucleotides encoding artificial or naturally-occurring mutants are also known. One skilled in the art can choose a polypeptide having activity to bind with synoviolin or P4HA1 from the various thus obtainable mutants. For example, when a certain protein is captured by an immobilized synoviolin, the protein is judged to have an activity to bind synoviolin. When a certain protein is captured by an immobilized P4HA1, the protein is judged to have an activity to bind synoviolin.

For example, fragment proteins of P4HA1 can be obtained by integrating a DNA fragment of P4HA1 cDNA into a suitable expression vector and expressing the DNA. Domains that are necessary for the synoviolin binding can be determined by verifying the binding activities of the above library of fragment proteins with synoviolin. Fragments of synoviolin comprising the thus-determined domain(s) can be used as a homolog of synoviolin in the methods of the present invention. Furthermore, P4HA1 counterparts derived from another species and which conserve an amino acid sequence constituting the domain, and polypeptides comprising a fragment sequence thereof can also be used as a homolog of P4HA1.

In the present invention, the methods for detecting the regulatory activity of a test compound on the ubiquitination effect of synoviolin are useful as methods of screening for compounds that regulate the ubiquitination effect of synoviolin through interference of the binding between synoviolin and a substrate. Namely, the present invention relates to methods of screening for a test compound with regulatory activity on the ubiquitination effect of synoviolin, comprising the following steps:
a) detecting regulatory activity of a test compound on the ubiquitination effect of synoviolin by the above detection methods of the regulatory activity of a test compound on the ubiquitination effect of synoviolin; and
b) selecting a test compound with a differential binding level of synoviolin and the substrate as compared with a control.

The methods for detecting regulatory activity on the ubiquitination effect of synoviolin according to the present invention are as described above. Using these methods, a test compound having the target activity can be selected by evaluating the activities of various test compounds and comparing the activities with a control. Any arbitrary compound having a verified level of the target activity can be used as the control in the present invention. For example, a compound which is known to have no target activity can be used as a negative control. Ubiquitination levels measured in the absence of a test compound may also be used as a negative control in comparison. By using a substance with a certain activity as a control, substances that have an activity exceeding the activity level can be screened.

Test compounds for use in the screening of the present invention are not specifically limited. For example, inorganic compounds, organic compounds, peptides, proteins, naturally-occurring or synthetic low-molecular compounds, naturally-occurring or synthetic high-molecular compounds, tissue or cell extracts, culture supernatants of microorganisms, and vegetable- or marine organism-derived natural components can be used as the test compounds. Expressed products of gene libraries or expressed cDNA libraries can also be used as the test compounds.

According to the screening methods of the present invention, a compound having regulatory activity on the synoviolin ubiquitination of P4HA1 can be selected. Such compounds include agents for treating rheumatoid arthritis or fibrosis. The "regulatory activity on ubiquitination effect" in the present invention includes stimulation and inhibition of the activity. Ubiquitination of a protein induces selective degradation of the protein by an ATP-dependent protease. Ubiquitination is assumed to eliminate abnormal proteins and unnecessary proteins in a living body. Consequently, the activity of prolyl 4-hydroxylase can be controlled by regulating the synoviolin ubiquitination of P4HA1.

Facilitation of the ubiquitination of P4HA1 increases the activity level of the enzyme. For example, the expression of synoviolin is increased in synovial cells of rheumatoid arthritis patients. As a result, the ubiquitination of P4HA1 is facilitated, and the activity level of the enzyme increases. A predicted mechanism thereof is that abnormal molecules are specifically degraded as a result of the P4HA1 ubiquitination, and the proportion of normal proteins increases. Examples of abnormal proteins are the following proteins:
- abnormally modified proteins
- proteins with abnormal folding.

Vice versa, when the ubiquitination of P4HA1 is inhibited, the accumulation of abnormal proteins advances and the prolyl 4-hydroxylase activity decreases. Abnormal proteins cause the decrease not only because of their enzymatic activities, but also because they work to inhibit the association between normal proteins and other subunits or the association between an enzyme molecule and a substrate.

Compounds having a regulatory effect on the synoviolin ubiquitination of P4HA1 are useful as agents for regulating the synoviolin ubiquitination of substrates. Namely, the present invention provides agents for regulating the synoviolin ubiquitination of a substrate, wherein the agents comprise as an active ingredient a compound that can be selected by the methods of the present invention. The agents for regulating the ubiquitination according to the present invention are useful for treating and/or preventing diseases caused by abnormality in the prolyl 4-hydroxylase activity. Prolyl 4-hydroxylase is an enzyme responsible for collagen metabolism. Accordingly, diseases accompanied by abnormality in collagen synthesis can be treated or prevented by regulating the ubiquitination by synoviolin.

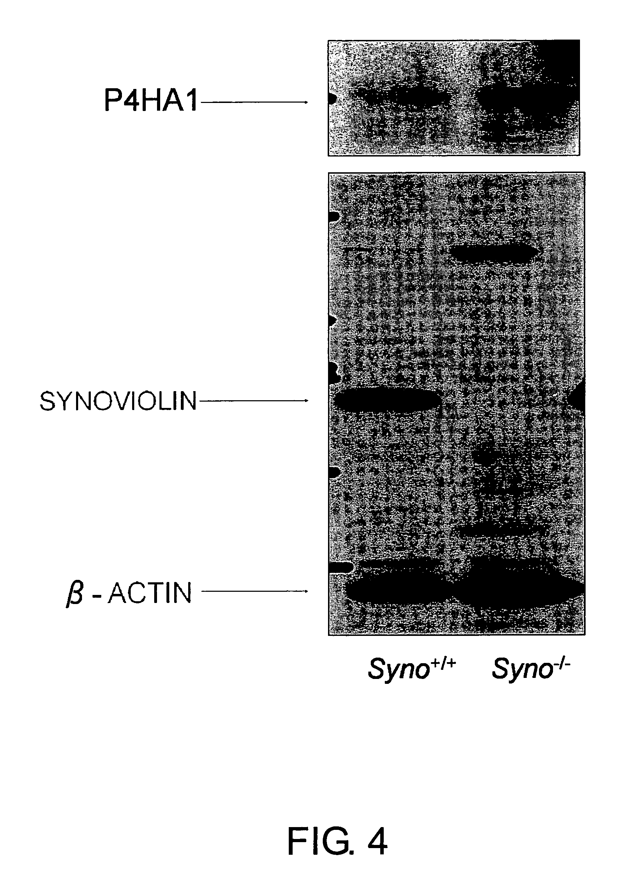

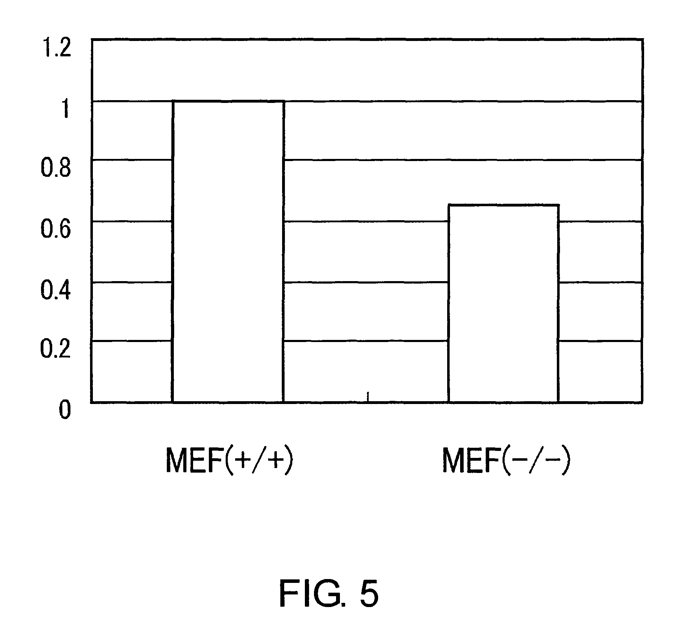

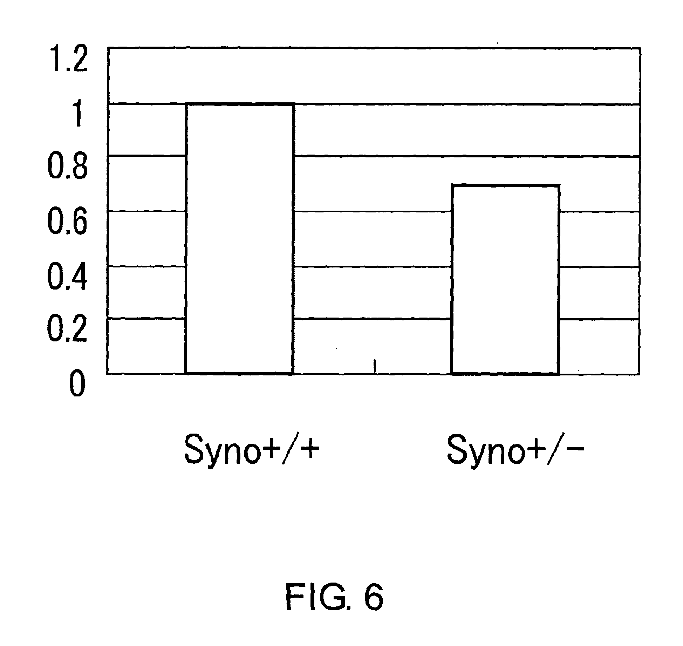

In fact, the present inventors have experimentally confirmed that murine cells in which synoviolin has been deleted through genetic engineering show decreased prolyl 4-hydroxylase activity (Fig. 6) and decreased collagen production (Fig. 5) as compared with normal cells. There is no significant difference in the protein amount of P4HA1 between these cells and the normal cells (Fig. 4). That is, the ubiquitination of P4HA1 by synoviolin is considered to induce the increase of prolyl 4-hydroxylase activity. Consequently, excessive collagen production can be suppressed by inhibiting the effect of synoviolin. Alternatively, collagen production can be stimulated by potentiating the effect of synoviolin.

More specifically, synoviolin inhibitors discovered using the effect on P4HA1 ubiquitination as an index are useful for treating and/or preventing diseases or pathological conditions caused by the accumulation of fibrocytes. For example, pulmonary fibrosis or hepatic fibrosis can be listed as examples of diseases or pathological conditions caused by the accumulation of fibrocytes (Kivirikko KI., Ann Med., 1993, Apr; 25(2):113-26).

Fibrosis in the present invention refers to a disease with pathological conditions in which the formation and accumulation of fibers increase as a response to the repair and damage of tissues. The above-mentioned pulmonary fibrosis and hepatic fibrosis are representative diseases of fibrosis. A possibility has been pointed out that various mechanisms are involved in the causes of these diseases. It is common to both cases that tissue fibrosis impairs the tissue function. For example, respiration function is impaired by pulmonary fibrosis. Hepatic fibrosis inhibits hepatic bloodstream and causes liver function disorders. Consequently, symptoms of these diseases can be treated, cured, or prevented if fibrosis can be improved.

The progression of fibrosis such as hepatic fibrosis is known to be suppressed by inhibiting collagen production (Kivirikko KI., Ann Med., 1993 Apr; 25(2): 113-26).

Methods of screening for a compound for treating or preventing fibrosis according to the present invention can be conducted by the same procedure as in the above-mentioned methods of screening for a compound which regulates the synoviolin ubiquitination of P4HA1.

Consequently, the present invention provides methods of screening for a compound for treating or preventing fibrosis, comprising the steps of:
a) incubating synoviolin or a homolog thereof and a substrate in the presence of a test compound under enabling conditions for substrate ubiquitination,
a') contacting either (i) a substrate or (ii) synoviolin or a homolog thereof with a test compound and incubating with the other of (i) and (ii) under enabling conditions for substrate ubiquitination, or
a") incubating a substrate with synoviolin or a homolog thereof under enabling conditions for substrate ubiquitination, and then contacting them with a test compound;
b) measuring level of substrate ubiquitination after any one of steps a), a'), and a"); and
c) selecting a test compound with a substrate ubiquitination level lower than a ubiquitination level measured in the absence of the test compound as a compound for treatment or prevention of fibrosis,
wherein the substrate is prolyl 4-hydroxylase α subunit or a homolog thereof.

Alternatively, the present invention provides methods of screening for a compound for treating or preventing fibrosis, comprising the steps of:
a) incubating in the presence of a test compound under enabling conditions for binding of the synoviolin or a homolog thereof and the substrate,
a') contacting either (i) a substrate or (ii) synoviolin or a homolog thereof with a test compound and incubating under enabling conditions for binding of the synoviolin and the substrate, or
a") incubating under enabling conditions for binding of the synoviolin or a homolog thereof and the substrate and contacting them with a test compound;
b) measuring the level of binding between the substrate and synoviolin or a homolog thereof after any one of steps a), a'), and a"); and
c) selecting a test compound with a binding level between the substrate and the synoviolin or a homolog thereof lower than a binding level measured in the absence of the test compound as a compound for treatment or prevention of fibrosis,
wherein the substrate is prolyl 4-hydroxylase α subunit or a homolog thereof.

Overproduction of collagen in rheumatism is one of the representative pathological conditions of rheumatism. Accordingly, controlling the synoviolin regulation of P4HA1 activity and inhibiting collagen production is effective as a rheumatism treatment strategy (Kivirikko KI. Ann Med. 1993 Apr; 25(2): 113-26.).

Consequently, the present invention provides methods of screening for a compound for treating or preventing rheumatoid arthritis, comprising the steps of:
a) incubating synoviolin or a homolog thereof and a substrate in the presence of a test compound under enabling conditions for substrate ubiquitination,
a') contacting either (i) a substrate or (ii) synoviolin or a homolog thereof with a test compound and incubating with the other of (i) and (ii) under enabling conditions for substrate ubiquitination, or
a") incubating a substrate and synoviolin or a homolog thereof under enabling conditions for substrate ubiquitination and contacting them with a test compound;
b) measuring level of substrate ubiquitination after any one of steps a), a'), and a"); and
c) selecting a test compound with a substrate ubiquitination level lower than a ubiquitination level measured in the absence of the test compound as a compound for treatment or prevention of rheumatoid arthritis,
wherein the substrate is prolyl 4-hydroxylase α subunit or a homolog thereof.

The present invention relates to methods of screening for a compound for treating or preventing rheumatoid arthritis, comprising the steps of:
a) incubating in the presence of a test compound under enabling conditions for binding of the synoviolin or a homolog thereof and the substrate;
a') contacting either (i) a substrate or (ii) synoviolin or a homolog thereof with a test compound and incubating under enabling conditions for binding of the synoviolin and the substrate, or
a") incubating under enabling conditions for binding of the substrate and the synoviolin or a homolog thereof and contacting them with a test compound;
b) measuring the level of binding between the substrate and the synoviolin or a homolog thereof after any one of steps a), a'), and a"); and
c) selecting a test compound with a binding level between the substrate and the synoviolin or a homolog thereof lower than a binding level measured in the absence of the test compound as a compound for treatment or prevention of rheumatoid arthritis,
wherein the substrate is prolyl 4-hydroxylase α subunit or a homolog thereof.

At the same time, synoviolin-activating agents discovered using the effect on P4HA1 ubiquitination as an index are useful in collagen supplementation therapies. For example, collagen which decreases from aging, UV ray irradiation, stress, or such can be supplemented by administering a synoviolin-activating agent.

In addition, the present invention relates to kits for detecting regulatory activity on the ubiquitination effect of synoviolin and kits for screening for a compound for treating or preventing fibrosis and rheumatoid arthritis, each of which comprises the following components. The following components (1) and (2) which are necessary for the kits of the present invention can be obtained in the above-mentioned manner.
(1) Synoviolin or a homolog thereof, and
(2) P4HA1 or a homolog thereof
   P4HA1 or a homolog thereof in the kits of the present invention may be labeled in advance. Alternatively, the kits may further comprise a means for labeling. For example, the kit can further comprise a labeled antibody that recognizes and binds P4HA1 or a homolog thereof.
   The kits of the present invention can further comprise the following components. These components are also as mentioned above.
(3) Ubiquitin activating enzyme,
(4) Ubiquitin transferase,
(5) Ubiquitin, and
(6) Adenosine triphosphate (ATP)

Of the above constitutive components, ubiquitin may be labeled in advance. Alternatively, the kits can further comprise a means for labeling ubiquitin. For example, a labeled antibody that recognizes and binds ubiquitin can be used.

P4HA1 that constitute the kits of the present invention can be bound to a solid phase such as beads. Particularly, beads comprising a liquid scintillant are advantageous, as the binding between ³²P-labeled ubiquitin and P4HA1 can be easily detected. Consequently, kits comprising P4HA1 bound to liquid scintillant-containing beads, and radioactively labeled ubiquitin are preferred as the kits of the present invention.

The kits of the present invention can further comprise a vessel and/or a medium for cell culturing. The kits of the present invention can be used for implementing the above-mentioned methods of the present invention.

Compounds identified by the testing or screening methods of the present invention are candidates of pharmaceutical agents for diseases involving synoviolin and prolyl 4-hydroxylase, and can be used for the prevention or treatment thereof. Fibrosis and rheumatoid arthritis, for example, are examples of such diseases. These compounds can be formulated into pharmaceutical compositions by appropriately incorporating other solutes and solvents in addition to the active ingredient. When a compound isolated by the screening methods of the present invention is used as a medicament, the isolated compound can not only be directly administered to a patient but also administered as a pharmaceutical composition prepared by formulating the isolated compound according to known pharmaceutical procedures.

For example, the compound can be administered by formulating in combination with pharmacologically acceptable carriers or vehicles such as sterile water, physiological saline, vegetable oils, emulsifying agents, and suspending agents, as appropriate. The pharmaceutical compositions of the present invention can be in any form such as aqueous solutions, tablets, capsules, troches, buccal tablets, elixirs, suspensions, syrups, nasal drops, and inhalants. Content of the compound may be determined as appropriate. Administration to a patient can be generally conducted according to techniques known by one skilled in the art, for example, intraarterial injection, intravenous injection, hypodermic injection, oral administration, and intraarticular injection.

The dosage varies with the body weight and age of the patient, administration method, symptoms, and such, but one skilled in the art can appropriately choose a suitable dosage. While a general dosage varies depending on the effective blood concentration and metabolic rate of an agent, the daily maintenance dose is considered to be about 0.1 mg/kg to about 1.0g/kg, preferably about 0.1 mg/kg to about 10 mg/kg, and more preferably about 0.1 mg/kg to about 1.0 mg/kg. The administration can be conducted in one to several times. If the compound can be encoded by a polynucleotide, gene therapy can be conducted by incorporating the polynucleotide into a gene therapy vector.

The prior art documents cited in the present specification are hereby incorporated by reference in their entirety.

### Brief Description of the Drawings

Fig. 1 is a diagram showing the construction structure of a bait used in the screening using a yeast two-hybrid system.
Fig. 2 is an autoradiograph showing the binding between [³²P]-labeled Flag-P4HAl fused protein and GST-Syno dTM and GST-Syno RING fused proteins.
Fig. 3 is an autoradiograph showing the results of ubiquitin assay of GST protein or GST-fused P4HA1 protein by synoviolin proteins: Syno dTM, Syno dTM (C307S), Syno dTM (dRING), and Syno RING.
Fig. 4 is a photograph showing the amount of P4HA1 expression in different murine fetal fibroblasts determined by Western blotting.
Fig. 5 is a graph showing the collagen contents in culture supernatants of different murine fetal fibroblasts, with the vertical axis indicating the ratio of collagen content (defining 0.09 µg collagen /mg protein as 1) and the horizontal axis indicating the type of murine cell.
Fig. 6 is a graph showing the prolyl 4-hydroxylase activity in different murine fetal fibroblasts, with the vertical axis indicating the ratio of ³H-H₂O production wherein the actual value of Syno+/+ is defined as 1, and the horizontal axis indicating the type of murine cell.
Fig. 7 shows micrographs (200 times) indicating synoviolin expression in pathological tissues with liver cirrhosis.
   Upper left: staining with an anti-synoviolin antibody
   Upper right: hematoxylin nuclear stain
   Lower left: staining with murine IgG (control)
Fig. 8 shows micrographs (200 times/right and 400 times/left) indicating synoviolin expression in pathological tissues with nodular fasciitis. The micrographs indicate staining with in the order from above, murine IgG, an anti-synoviolin antibody, and an anti-synoviolin antibody with a blocking peptide.

### Best Mode for Carrying Out the Invention

Next, the present invention will be specifically described with reference to Examples.

### [Example 1] Screening for P4HA1 by yeast two-hybrid method

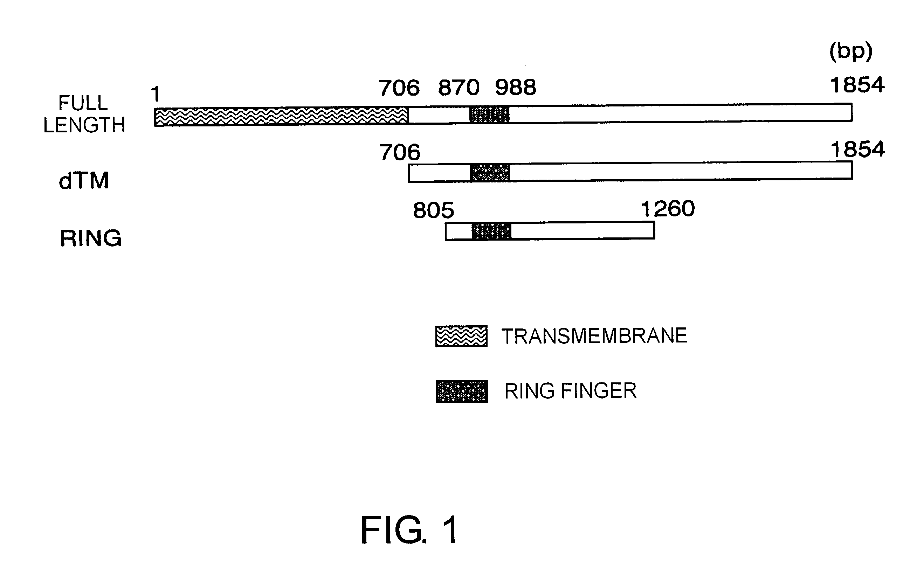

Screening for synoviolin substrate proteins was conducted by a yeast transformation method (Pro. Natl. Aca. Sci. USA., 88: 9578-9582, 1991) using the Clontech MATCHMAKER Two-Hybrid System as a yeast two-hybrid system. The termini of synoviolin cDNA at 706 bp to 1854 bp and 805 bp to 1260 bp were modified with EcoR I/Xho I and the cDNAs were inserted into the EcoR I/Xho I site of pGBT9 vector. Herein below, fragments of synoviolin at 706 bp to 1854 bp and at 805 bp to 1260 bp are referred to as Syno dTM and Syno RING, respectively (Fig. 1).

A library (pACT2-Y) prepared by inserting a human cartilage-derived cDNA into pACT2 vector was used in the screening for synoviolin substrate proteins. After applying a heat shock at 42°C for 15 minutes, pGBT9-Syno dTM or pGBT9-Syno RING (2.0 µg), and pACT2-Y (20 µg) were introduced into yeast Y190 strain. After washing the vector-introduced yeast Y190 with Tris-EDTA (pH 7.5) buffer (TE buffer), a solution of the TE buffer-diluted yeast Y190 was spread on a SD-Trp-Leu-His plate and incubated at 30°C for 10 days. A β-galactosidase filter assay using 0.5 mg/ml X-gal as a substrate was conducted on developed colonies, and a positive clone was detected.

The positive clone was cultured in a SD-Leu-His medium at 30°C for 10 dayswhile shaking, and a yeast DNA was extracted using the alkali-SDS method (Methods Enzymol., 194:169-182, 1991). The extracted yeast DNA was transformed into *Escherichia coli* HB101 strain, spread on a M9 plate (-Leu), and cultured at 37°C for 2 days. A developed colony was cultured in a LB medium supplemented with 20 µg/ml ampicillin at 37°C for 16 hours while shaking, and a plasmid DNA was extracted by the alkali-SDS method. The human cartilage-derived cDNA fragment which had been inserted into the extracted plasmid DNA was analyzed using the BigDye Terminator Cycle Sequencing system from Applied Biosystems.

The sequence analysis results by BLAST (http://www.ncbi.nlm.nih.gov/BLAST/) showed that an α subunit of prolyl 4-hydroxylase, a key enzyme in collagen production for hydroxylation of the proline residue of procollagen (procollagen-proline, 2-oxoglutarate 4-dioxygenase (proline 4-hydroxylase), alpha polypeptide I (ACCESSION No. XM_032511P4HA1) was obtained.

### [Example 2] Binding between synoviolin and P4HA1

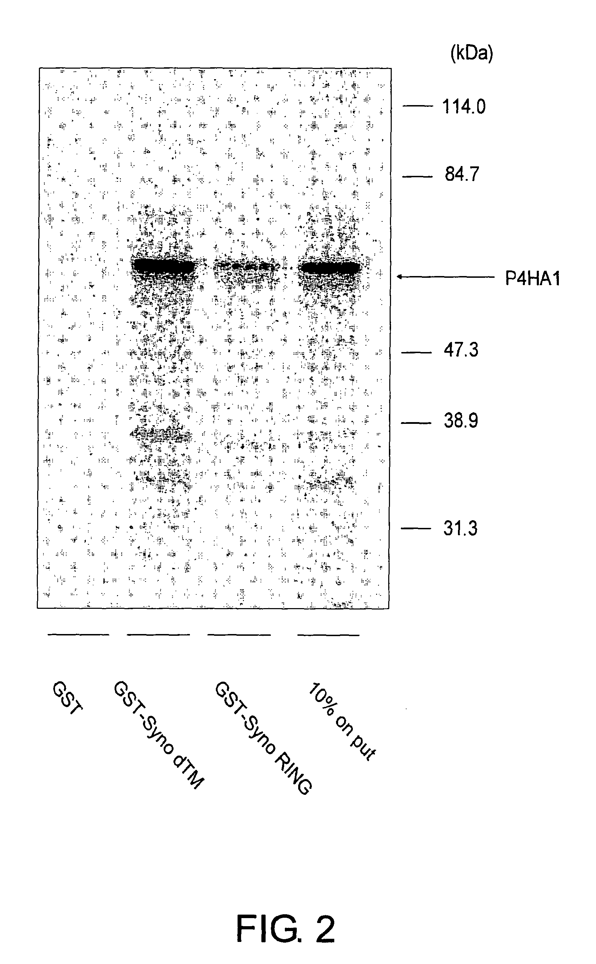

A [³⁵S]-labeled Flag-P4HA1 fused protein was prepared using the TNT-coupled Translation System (Promega Corporation) and pcDNA3-Flag-P4HA1. The fused protein was added, together with GST-Syno dTM, GST-Syno RING and GST protein, to a binding buffer containing 20 mM HEPES (pH 7.9), 100 mM NaCl, 1 mM EDTA, 0.05% Tween, 5% glycerol, 1 mM DTT, 0.2 mM NaVO₄, 5 mM NaF, and 1 mM PMSF and reacted at 4°C for 16 hours. The resulting reaction product was developed over SDS-PAGE and its radioactivity was detected with an image analyzer (BAS2000, Fujix). The binding of GST-Syno dTM and GST-Syno RING fusion protein with [³⁵S]-labeled pcDNA3-Flag-P4HA1 was observed. In contrast, no binding was found between the control GST protein and pcDNA3-Flag-P4HA1 (Fig. 2). These results show that synoviolin binds to P4HA1.

### [Example 3] Identification of substrate for synoviolin as ubiquitin ligase (E3)

Ubiquitin covalently binds to a target protein (substrate) by the action of a ubiquitin-related enzyme system comprising an activating enzyme (E1), a binding enzyme (E2) and a ligase (E3). By repeating this reaction, a polyubiquitin chain is formed, and this acts as a marker for the proteasome degradation of substrate. E3 is the most important enzyme in substrate selection. Identification of a substrate protein for synoviolin is considered to be essential for determining the signaling pathway in which synoviolin participates. A protein that binds with synoviolin obtained by screening using the yeast two-hybrid method is a candidate substrate for synoviolin ubiquitin ligase (E3).

Consequently, an *in vitro* investigation using a ubiquitin ligase activity measurement system was made. cDNAs comprising Syno dTM, Syno RING, Syno dTM (C307S) with 307th cysteine to serine substitution, Syno dTM (dRING) with 291 st to 329th amino acid deletion, and the whole region of P4HA1 were inserted into GST-fused protein vectors (pGEX 4T-1), and fused proteins were produced in *Escherichia coli.* Fused proteins obtained from solubilized supernatants of *Escherichia coli* were bound to Glutathione Sepharose™ 4B beads (Amersham Biosciences).

Next, the GST moiety of each of the synoviolin-fused protein beads was removed by thrombin (a type of protease) to thereby yield synoviolin proteins. His-ubiquitin protein previously inserted into the PKA site was labeled with [³²P-γ] ATP. E1 (yeast-derived), E2 (UbcH5c), ATP, [³²P-γ]-labeled ubiquitin, GST fused P4HA1 protein beads, and each synoviolin protein were admixed and reacted at 37°C for 60 minutes. After reaction, each sample was admixed with 4x SDS-PAGE buffer, boiled for 5 minutes, and developed over 10% SDS-PAGE.

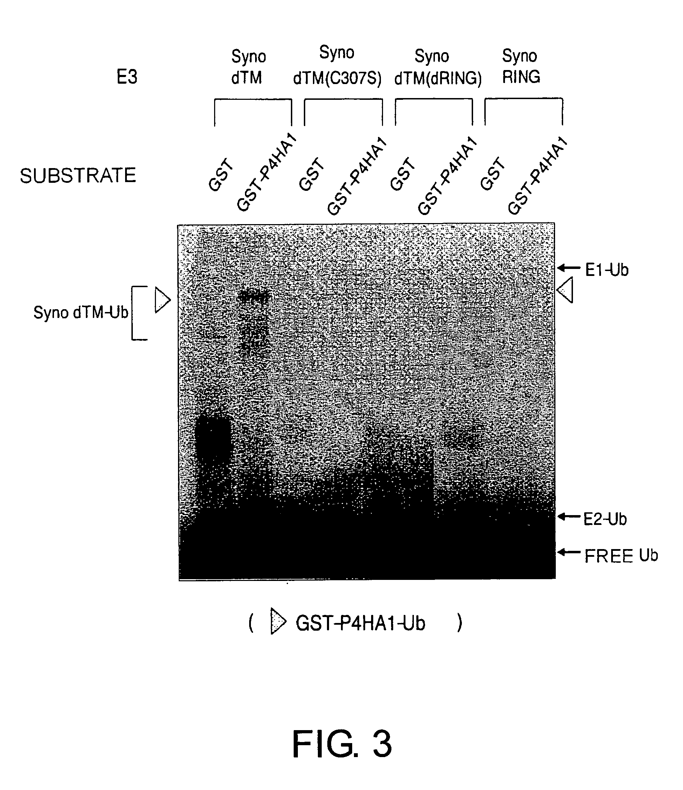

Imaging of the developed gels was conducted using an image analyzer (BAS2000, Fujix), and ubiquitinated bands were detected. The results showed that P4HA1 was ubiquitinated by Syno dTM and Syno RING (Fig. 3). However, P4HA1 was not ubiquitinated by Syno dTM (C307S) and Syno dTM (dRING). This revealed that P4HA1 is a substrate of synoviolin.

### [Example 4] Synoviolin and collagen production

### Levels of P4HA1 expression and collagen production in various murine fetal fibroblasts (MEFs)

Fetal fibroblasts (MEFs) were plated at 2x 10⁵ cells, cultured for 3 days, and then recovered. The P4HA1 expression level was quantitatively determined by Western blotting using an anti-P4HA1 antibody (anti-hPH(α), purified IgG, Daiichi Fine Chemical Co., Ltd.). Simultaneously, the expression levels of synoviolin and β actin were compared using an anti-synoviolin antibody (monoclonal antibody, 10 Da) and an anti-β-actin antibody (monoclonal anti-β-actin, Clone AC-15, Sigma). Collagen was quantitatively determined using the Sircol TM Soluble Collagen Assay Kit (Biocolor Ltd.). The data were processed by defining the collagen content in MEF (syno +/+) cells (0.09 µg collagen/mg protein) as 1.

As a result, the P4HA1 protein level of synoviolin-deleted mouse (syno -/-) MEFs was substantially the same as that of wild type mouse (syno +/+) MEFs (Fig. 4). In contrast, the collagen content of the culture supernatants of syno -/- MEFs was found to be lower than that of syno +/+ (Fig. 5). These two findings indicate a possibility that synoviolin controls the quality of prolyl 4-hydroxylase protein in cells by ubiquitinating P4HA1.

### Comparison of prolyl 4-hydroxylase activities among various MEFs

The prolyl 4-hydroxylase activity was measured with reference to the following articles.
1) Gribble TJ, Comstock JP, Udenfriend S., "Collagen chain formation and peptidyl proline hydroxylation in monolayer tissue cultures of L-929 fibroblasts.", Arch Biochem Biophys., 1969, Jan;129(1):308-16.
2) Margolis RL, Lukens LN., "The role of hydroxylation in the secretion of collagen by mouse fibroblasts in culture. Arch Biochem Biophys.", 1971, Dec;147(2):612-8.
3) Methods enzymol., Vol. 82 (1982) p247-265.

To the medium of normal synovial cells (i.e., collagen-producing cells), α,α'-dipyridyl was added to terminate the hydroxylation reaction by cellular hydroxylase, and then [2,3-³H] proline was then added to the culture supernatant, and synthesized ³H-protocollagen was extracted. Lysates comprising P4H were recovered from syno -/- MEF cultured cells and syno +/+ MEF cultured cells, and the activities of hydroxylating ³H-protocollagen (amount of ³H-H₂O production) were measured.

As a result, the amounts of ³H-H₂O production were 39.28 cpm/µg total cell extract in Syno+/+ and 27.58 cpm/µg total cell extract in Syno-/-. The amount ratios of ³H-H₂O production by defining the actual value of Syno+/+ as 1 are shown in Fig. 6, indicating that the enzyme activity of P4H in syno -/- MEFs is lower than that in syno +/+ MEFs (Fig. 6).

### [Example 5] Synoviolin expression in pathological tissues with liver cirrhosis and nodular fasciitis

To identify the site of synoviolin expression in diseases accompanied by fibrosis, tissue immunostaining with an anti-synoviolin antibody was conducted using pathological tissues of liver cirrhosis and nodular fasciitis.

The pathological tissues of liver cirrhosis and nodular fasciitis were obtained with informed consent. The tissues were fixed with formalin according to conventional procedures, embedded in paraffin, sliced with a microtome, and used as tissue specimens. The tissue sections, from which paraffin was fully removed by xylene treatment (three times for five minutes each), were dipped into a 6-level descending series (10% decrease in each) of alcohol solutions from 100% ethyl alcohol to 50% ethyl alcohol solution, and blocked with 1% bovine serum albumin (BSA) for 30 minutes.

After the completion of blocking, the tissues were reacted with a PBS-diluted primary antibody solution (anti-synoviolin monoclonal antibody) comprising 1% BSA at room temperature for 60 minutes. Next, the tissues were washed five times in PBS for 5 minutes and then reacted with a secondary HRP-labeled anti-mouse immunoglobulin antibody diluted with 1% BSA solution at room temperature for 30 minutes. After the completion of the reaction, the tissues were washed five times in PBS for 5 minutes and further reacted with a coloring reagent (3, 3'-diaminobenzidine tetrahydrochloride). After a suitable coloration was achieved, the tissues were washed with water and observed under a microscope.

Additionally, the tissues were subjected to hematoxylin nuclear staining as a counterstain and observed under a microscope. Furthermore, as a control, a sample reacted with a blocking peptide comprising an epitope amino acid sequence of the anti-synoviolin monoclonal antibody used in the staining was observed under the microscope.

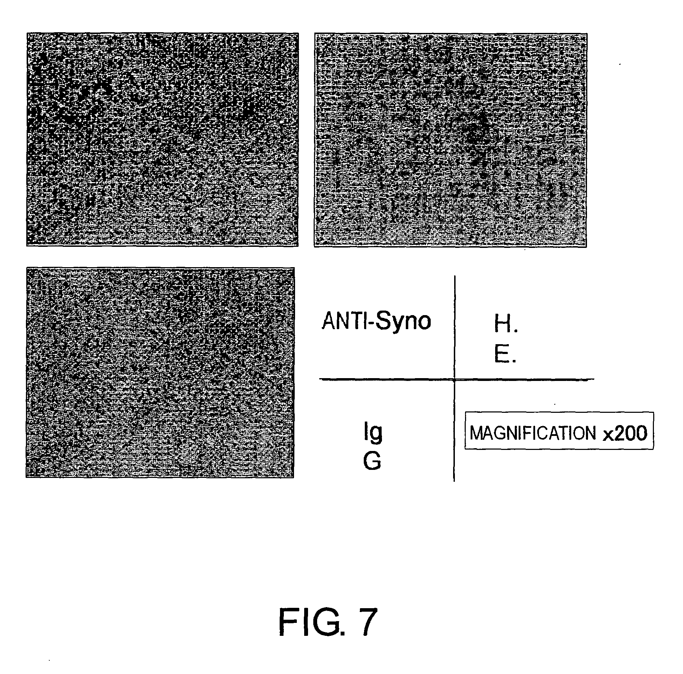

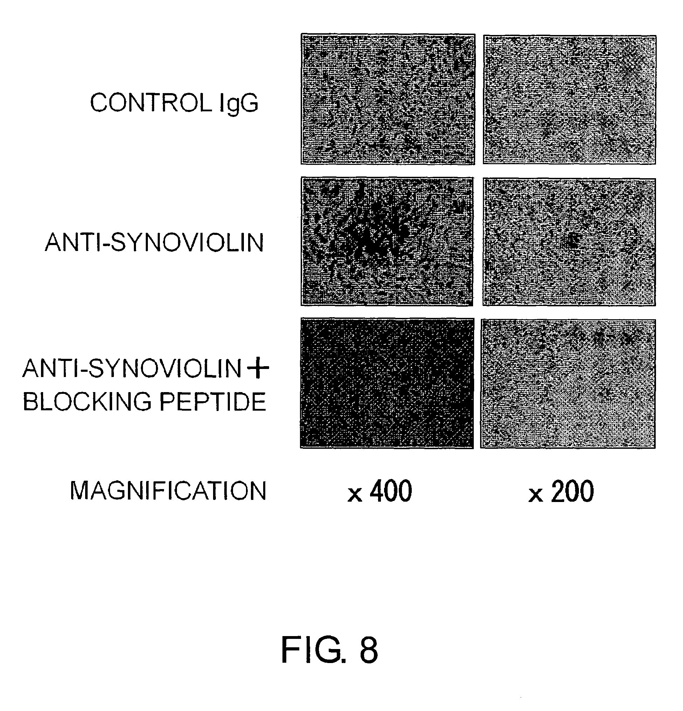

As a result, high synoviolin expression was observed in the hepatic parenchymal cells of liver cirrhosis tissues, particularly, in portions with high cell proliferation (Fig. 7). In nodular fasciitis tissues which are featured by the tumor-like proliferation of fascial fibroblasts, high synoviolin expression was also observed in tubercle portions with high cell proliferation (Fig. 8).

### [Example 6] Methods of screening for compounds that inhibit the binding between synoviolin and P4HA1

Methods of high-throughput screening (HTS) for compounds that inhibit the binding between synoviolin and its substrate P4HA1 were investigated. A system was designed in which synoviolin was expressed as a His tag-fused protein and P4HA1 was expressed as a GST-fused protein. In this system, when synoviolin was bound to P4HA1, energy transfer occurred between a XL665-labeled anti-His antibody and a Eu(K)-labeled anti-GST antibody and fluorescence was then detected.

### 1) Preparation of MBP-Syno dTM-His

MBP synoviolin dTM-Hisx₁₂ comprises a MBP-fused synoviolin that lacks the transmembrane segment (TM) (synoviolin dTM). MBP synoviolin dTM-Hisx₁₂ was prepared by producing the fused protein in *Escherichia coli* and purifying the same with a Ni-NTA Column (QIAGEN).

### 2) Preparation of His-E2

His-E2 was obtained by inserting a cDNA comprising the whole region of UbcH5c into a His tag-fused protein vector (pET), producing a fused protein in *Escherichia coli,* and purifying the produced fused protein with a Ni-NTA Column (QIAGEN).

### 3) Preparation of GST P4HA1

A cDNA comprising the whole region of P4HA1 was inserted into a GST fused protein vector (pGEX 4T-1), and a fused protein was produced in *Escherichia coli.* GST-P4HA1 was prepared by obtaining the fused protein from a solubilized supernatant of *Escherichia coli* and purifying the same using Glutathione Sepharose™ 4B beads (Amersham Bioscience).

### 4) Measurement of inhibitory activity on binding between synoviolin and P4HA1

A standard reaction mixture comprising 60 ng MBP-synoviolin-His, 10 ng GST-P4HA1, 1 mM DTT, 1 mM EDTA, 50 mM NaCl, 0.1% BSA, 0.3% DMSO, and a test compound in 15 µL of 50 mM HEPES (pH 7.5) solution was prepared using a 384-well plate. The reaction was initiated by adding 5 µL of a 50 mM HEPES solution comprising GST-P4HA1. After a 30 minute reaction at room temperature, 5 µL of a 50 mM HEPES solution comprising an Eu(K)-labeled anti-GST antibody (20000 B counts, CIS bio international), a XL665-labeled anti-His antibody (0.025 µg, CIS bio international), and 1 M KF was added. The mixture was left to stand overnight at room temperature, and the fluorescence was measured using RUBYstar™.

According to an investigation of the inhibitory activity, a partial peptide of the partially modified synoviolin (SEQ ID NO: 5) inhibits the binding between synoviolin and P4HA1 at an IC₅₀ of 167 µg/ml.

### [Example 7] Methods of screening for compounds that inhibit synoviolin activity of ubiquitinating P4HA1

### 1) Method of screening for compounds that inhibit the ubiquitination activity using energy transfer of XL665-Eu(K) (HTRF method)

A method of high-throughput screening (HTS) for compounds that inhibit the synoviolin activity of ubiquitinating P4HA1 was investigated. A system was designed in which ubiquitination by Eu(K)-labeled ubiquitin was conducted using P4HA1 expressed as a GST-fused protein as a substrate. When P4HA1 underwent Eu(K)-labeled ubiquitination, energy transfer occurred between an XL665-labeled anti-GST antibody and an Eu(K)-labeled ubiquitin, and fluorescence was detected.

A standard reaction mixture comprising 60 ng MBP-synoviolin-His, 15 ng GST-P4HA1, 15 ng E1 (Boston Biochem), 200 ng His-E2, 6.25 nM Eu(K)-labeled ubiquitin (CIS bio international), 5 mM MgCl₂, 2 mM ATP, 1 mM DTT, 0.1% BSA, 0.3% DMSO, and a test compound in 15 µL of a 20 mM HEPES (pH 7.5) solution was prepared using a 384-well plate. After a 30 minute reaction at 37°C, 5 µL of 0.5 M EDTA was added to terminate the reaction. To this was added 5 µL of 20 mM HEPES solution containing an XL665-labeled anti-His antibody (0.025 µg) and 1 M KF, and the mixture was left to stand overnight at room temperature, and the fluorescence was measured using RUBYstar™.

A partial peptide of the partially modified synoviolin (SEQ ID NO: 5) inhibited the ubiquitination of P4HA1 at a concentration 20 times lower than that for inhibition of the binding.

### 2) Method of screening for compounds that inhibit ubiquitination activity using ELISA

A 96-well ELISA plate was adsorbed with an anti-GST antibody at 4°C overnight and then blocked with 5% BSA/PBS solution at room temperature until it was used. The plate was washed twice before use with 300 µL of PBS containing 0.3% BSA (PBSA).

A standard reaction mixture comprising 80 ng MBP-synoviolin-His, 40 ng GST-P4HA1, 15 ng E1, 200 ng His-E2, 5 mM MgCl₂, 2 mMATP, 1 mM DTT, 0.1% BSA, 0.3% DMSO, and a test compound in 30 µL of 50 mM HEPES (pH 7.5) solution was prepared in a reaction tube. After a 30 minute reaction at room temperature, 70 µL of a solution containing 70 mM EDTA and 50 mM HEPES was added to terminate the reaction. This (30 µl) was added to a plate previously containing 70 µL PBSA and incubated at room temperature for 1 hour. After washing three times with 300 µL of PBSA, 100 µL of a mouse anti-ubiquitin antibody solution diluted 1:5000 in PBSA was added, and the mixture was incubated at room temperature for 1 hour. After three washes with 300 µL of PBSA, 100 µL of a peroxidase-labeled anti-mouse IgG solution diluted 1:10000 in PBSA was added, and the mixture was incubated at room temperature for 1 hour. After three washes with 300 µL of PBSA, 100 µL of TMB solution was added, and the mixture was incubated at room temperature. When a sufficient coloration was observed, 1 M phosphoric acid was added to terminate the reaction, and the optical density at 450 nm was measured.

A partial peptide of the partially modified synoviolin (SEQ ID NO: 5) inhibited the ubiquitination of P4HA1 at an IC₅₀ of 55 µg/ml.

### Industrial Applicability

Regulatory activities on the synoviolin ubiquitination of prolyl 4-hydroxylase α subunit can be evaluated according to the present invention. Additionally, methods of screening for compounds that have regulatory activity on the synoviolin ubiquitination of prolyl 4-hydroxylase α subunit are provided based on the methods of the present invention. Furthermore, the present invention provides methods of screening for compounds for treating or preventing rheumatoid arthritis.

The fact that synoviolin regulates the activity of prolyl 4-hydroxylase through ubiquitination of the α subunit is a novel finding first revealed by the present inventors. Prolyl 4-hydroxylase is a key enzyme in the collagen synthesis of the body. Consequently, compounds that can be selected by the methods of the present invention are useful for treating and/or preventing diseases caused by abnormalities in the activity of prolyl 4-hydroxylase. For example, fibrosis or rheumatoid arthritis caused by the accumulation of collagen can be treated or prevented by compounds that are obtainable according to the present invention.

## Claims

1. A method for detecting regulatory activity of a test compound on the ubiquitination effect of synoviolin, comprising the steps of:
a) incubating synoviolin or a homolog thereof and a substrate in the presence of a test compound under enabling conditions for substrate ubiquitination,
a') contacting either (i) a substrate or (ii) synoviolin or a homolog thereof with a test compound and incubating with the other of (i) and (ii) under enabling conditions for substrate ubiquitination, or
a") incubating a substrate and synoviolin or a homolog thereof under enabling conditions for substrate ubiquitination and contacting them with a test compound;
b) measuring level of substrate ubiquitination after any one of steps a), a'), and a"); and
c) detecting regulatory activity of the test compound on the synoviolin ubiquitination of the substrate when the substrate ubiquitination level differs from a ubiquitination level measured in the absence of the test compound,
wherein the substrate is prolyl 4-hydroxylase α subunit or a homolog thereof.

2. The method of claim 1, wherein the enabling conditions for substrate ubiquitination are provided by coexistence of the following components:
i) a ubiquitin activating enzyme;
ii) a ubiquitin transferase;
iii) ubiquitin; and
iv) adenosine triphosphate.

3. The method of claim 1, wherein the enabling conditions for substrate ubiquitination are provided by ubiquitinating the substrate in a cell expressing the substrate and the synoviolin or a homolog thereof.

4. The method of claim 1, wherein the ubiquitination level of the substrate is measured using any one of the following levels as an index:
A) level of ubiquitinated substrate;
B) level of unubiquitinated substrate; and
C) level of prolyl 4-hydroxylase α subunit bioactivity.

5. The method of claim 1, wherein the prolyl 4-hydroxylase α subunit or a homolog thereof is any one of the following polypeptides (a) to (e):
(a) a polypeptide encoded by the nucleotide sequence of SEQ ID NO: 1;
(b) a polypeptide comprising the amino acid sequence of SEQ ID NO: 2;
(c) a polypeptide that comprises an amino acid sequence with a substitution, deletion, insertion, and/or addition of one or plural amino acids in the amino acid sequence of SEQ ID NO: 2 and which can be ubiquitinated by synoviolin;
(d) a polypeptide that comprises an amino acid sequence encoded by a polynucleotide hybridizing under stringent conditions with a DNA comprising the nucleotide sequence of SEQ ID NO: 1 and which can be ubiquitinated by synoviolin; and
(e) a polypeptide that comprises an amino acid sequence having a homology of 70% or more with the amino acid sequence of SEQ ID NO: 2 and which can be ubiquitinated by synoviolin.

6. The method of claim 1, wherein the synoviolin or a homolog thereof is any one of the following polypeptides (A) to (E):
(A) a polypeptide encoded by the nucleotide sequence of SEQ ID NO: 3;
(B) a polypeptide comprising the amino acid sequence of SEQ ID NO: 4;
(C) a polypeptide that comprises an amino acid sequence with a substitution, deletion, insertion, and/or addition of one or plural amino acids in the amino acid sequence of SEQ ID NO: 4 and which has an activity of ubiquitinating a polypeptide comprising the amino acid sequence of SEQ ID NO: 2;
(D) a polypeptide which hybridizes under stringent conditions with a DNA comprising the nucleotide sequence of SEQ ID NO: 3 and that has an activity of ubiquitinating a polypeptide comprising the amino acid sequence of SEQ ID NO: 2; and
(E) a polypeptide which comprises an amino acid sequence having a homology of 70% or more with the amino acid sequence of SEQ ID NO: 4 and has an activity of ubiquitinating a polypeptide comprising the amino acid sequence of SEQ ID NO: 2.

7. A method of screening for a test compound having regulatory activity on the ubiquitination effect of synoviolin, comprising the steps of:
a) detecting regulatory activity of a test compound on the ubiquitination effect of synoviolin by the method of any one of claims 1 to 6; and
b) selecting a test compound with a different substrate ubiquitination level when compared with a control.

8. An agent for regulating the synoviolin ubiquitination of a substrate, comprising a compound obtainable by the method of claim 7 as an active ingredient.

9. A kit for detecting regulatory activity on the ubiquitination effect of synoviolin, comprising the following components:
(1) synoviolin or a homolog thereof; and
(2) prolyl 4-hydroxylase α subunit or a homolog thereof.

10. The kit of claim 9, further comprising the following components:
(3) a ubiquitin activating enzyme;
(4) a ubiquitin transferase;
(5) ubiquitin; and
(6) adenosine triphosphate.

11. A kit for detecting regulatory activity on the ubiquitination effect of synoviolin, comprising a cell expressing synoviolin or a homolog thereof, and prolyl 4-hydroxylase α subunit or a homolog thereof; and a means for measuring ubiquitination level of the prolyl 4-hydroxylase α subunit or a homolog thereof.

12. A method for detecting regulatory activity of a test compound on the ubiquitination effect of synoviolin, comprising the steps of:
a) incubating in the presence of a test compound under enabling conditions for binding of the synoviolin or a homolog thereof and the substrate,
a') contacting either (i) a substrate or (ii) synoviolin or a homolog thereof with a test compound and incubating under enabling conditions for binding of the synoviolin and the substrate, or
a") incubating under enabling conditions for binding of the substrate and the synoviolin or a homolog thereof and contacting them with a test compound;
b) measuring the level of binding between the substrate and the synoviolin or a homolog thereof after any one of steps a), a'), and a"); and
c) detecting regulatory activity of the test compound on the synoviolin ubiquitination of the substrate when the level of binding between the substrate and the synoviolin or a homolog thereof differs from a binding level measured in the absence of the test compound,
wherein the substrate is prolyl 4-hydroxylase α subunit or a homolog thereof.

13. The method of claim 12, wherein either the synoviolin or the substrate is bound to a solid phase or comprises a label capable of binding to a solid phase.

14. The method of claim 12, wherein the prolyl 4-hydroxylase α subunit or a homolog thereof is any one of the following polypeptides (a) to (e):
(a) a polypeptide encoded by the nucleotide sequence of SEQ ID NO: 1;
(b) a polypeptide comprising the amino acid sequence of SEQ ID NO: 2;
(c) a polypeptide which comprises an amino acid sequence with a substitution, deletion, insertion, and/or addition of one or plural amino acids in the amino acid sequence of SEQ ID NO: 2 and can bind to synoviolin;
(d) a polypeptide which comprises an amino acid sequence encoded by a polynucleotide hybridizing under stringent conditions with a DNA comprising the nucleotide sequence of SEQ ID NO: 1 and can bind to synoviolin; and
(e) a polypeptide which comprises an amino acid sequence having a homology of 70% or more with the amino acid sequence of SEQ ID NO: 2 and can bind to synoviolin.

15. The method of claim 12, wherein the synoviolin or a homolog thereof is any one of the following polypeptides (A) to (E):
(A) a polypeptide encoded by the nucleotide sequence of SEQ ID NO: 3;
(B) a polypeptide comprising the amino acid sequence of SEQ ID NO: 4;
(C) a polypeptide which comprises an amino acid sequence with a substitution, deletion, insertion, and/or addition of one or plural amino acids in the amino acid sequence of SEQ ID NO: 4 and can bind to a polypeptide comprising the amino acid sequence of SEQ ID NO: 2;
(D) a polypeptide which hybridizes under stringent conditions with a DNA comprising the nucleotide sequence of SEQ ID NO: 3 and that can bind to a polypeptide comprising the amino acid sequence of SEQ ID NO: 2; and
(E) a polypeptide which comprises an amino acid sequence having a homology of 70% or more with the amino acid sequence of SEQ ID NO: 4 and can bind to a polypeptide comprising the amino acid sequence of SEQ ID NO: 2.

16. A method of screening for a test compound having regulatory activity on the ubiquitination effect of synoviolin, comprising the steps of:
a) detecting regulatory activity of a test compound on the ubiquitination effect of synoviolin by the method of any one of claims 12 to 15; and
b) selecting a test compound with a different level of binding between synoviolin and the substrate when compared with a control.

17. An agent for regulating the synoviolin ubiquitination of a substrate, comprising a compound obtainable by the method of claim 16 as an active ingredient.

18. A method of screening for a compound for treatment or prevention of fibrosis, comprising the steps of:
a) incubating synoviolin or a homolog thereof and a substrate in the presence of a test compound under enabling conditions for substrate ubiquitination,
a') contacting either (i) a substrate or (ii) synoviolin or a homolog thereof with a test compound and incubating with the other of (i) and (ii) under enabling conditions for substrate ubiquitination, or
a") incubating a substrate with synoviolin or a homolog thereof under enabling conditions for substrate ubiquitination, and then contacting them with a test compound;
b) measuring level of substrate ubiquitination after any one of steps a), a'), and a"); and
c) selecting a test compound with a substrate ubiquitination level lower than a ubiquitination level measured in the absence of the test compound as a compound for treatment or prevention of fibrosis,
wherein the substrate is prolyl 4-hydroxylase α subunit or a homolog thereof.

19. The method of claim 18, wherein the enabling conditions for substrate ubiquitination are provided by coexistence of the following ingredients:
i) a ubiquitin activating enzyme;
ii) a ubiquitin transferase;
iii) ubiquitin; and
iv) adenosine triphosphate.

20. The method of claim 18, wherein the enabling conditions for substrate ubiquitination are provided by ubiquitinating the substrate in a cell expressing the substrate and the synoviolin or a homolog thereof.

21. The method of claim 18, wherein the ubiquitination level of the substrate is measured using any one of the following levels as an index:
A) level of ubiquitinated substrate;
B) level of unubiquitinated substrate; and
C) level of prolyl 4-hydroxylase α subunit bioactivity.

22. The method of claim 18, wherein the prolyl 4-hydroxylase α subunit or a homolog thereof is any one of the following polypeptides (a) to (e):
(a) a polypeptide encoded by the nucleotide sequence of SEQ ID NO: 1;
(b) a polypeptide comprising the amino acid sequence of SEQ ID NO: 2;
(c) a polypeptide which comprises an amino acid sequence with a substitution, deletion, insertion, and/or addition of one or plural amino acids in the amino acid sequence of SEQ ID NO: 2 and can be ubiquitinated by synoviolin;
(d) a polypeptide which comprises an amino acid sequence encoded by a polynucleotide hybridizing under stringent conditions with a DNA comprising the nucleotide sequence of SEQ ID NO: 1 and can be ubiquitinated by synoviolin; and
(e) a polypeptide which comprises an amino acid sequence having a homology of 70% or more with the amino acid sequence of SEQ ID NO: 2 and can be ubiquitinated by synoviolin.

23. The method of claim 18, wherein the synoviolin or a homolog thereof is any one of the following polypeptides (A) to (E):
(A) a polypeptide encoded by the nucleotide sequence of SEQ ID NO: 3;
(B) a polypeptide comprising the amino acid sequence of SEQ ID NO: 4;
(C) a polypeptide which comprises an amino acid sequence with a substitution, deletion, insertion, and/or addition of one or plural amino acids in the amino acid sequence of SEQ ID NO: 4 and has an activity of ubiquitinating a polypeptide comprising the amino acid sequence of SEQ ID NO: 2;
(D) a polypeptide which hybridizes under stringent conditions with a DNA comprising the nucleotide sequence of SEQ ID NO: 3 and that has an activity of ubiquitinating a polypeptide comprising the amino acid sequence of SEQ ID NO: 2; and
(E) a polypeptide which comprises an amino acid sequence having a homology of 70% or more with the amino acid sequence of SEQ ID NO: 4 and has an activity of ubiquitinating a polypeptide comprising the amino acid sequence of SEQ ID NO: 2.

24. A pharmaceutical composition for treating and/or preventing fibrosis, comprising a compound selectable by the method of any one of claims 18 to 23 as an active ingredient.

25. A kit for screening for a compound for treatment or prevention of fibrosis, comprising the following components:
(1) synoviolin or a homolog thereof; and
(2) prolyl 4-hydroxylase α subunit or a homolog thereof.

26. The kit of claim 25, further comprising the following components:
(3) a ubiquitin activating enzyme;
(4) a ubiquitin transferase;
(5) ubiquitin; and
(6) adenosine triphosphate.

27. A kit for screening for a test compound for treatment or prevention of fibrosis, comprising a cell expressing synoviolin or a homolog thereof, and prolyl 4-hydroxylase α subunit or a homolog thereof; and a means for measuring the ubiquitination level of the prolyl 4-hydroxylase α subunit or a homolog thereof.

28. A method of screening for a compound for treatment or prevention of fibrosis, comprising the steps of:
a) incubating in the presence of a test compound under enabling conditions for binding of the synoviolin or a homolog thereof and the substrate,
a') contacting either (i) a substrate or (ii) synoviolin or a homolog thereof with a test compound and incubating under enabling conditions for binding of the synoviolin and the substrate, or
a") incubating under enabling conditions for binding of the substrate and the synoviolin or a homolog thereof and contacting them with a test compound;
b) measuring the level of binding between the substrate and synoviolin or a homolog thereof after any one of steps a), a'), and a"); and
c) selecting a test compound with a binding level between the substrate and the synoviolin or a homolog thereof lower than a binding level measured in the absence of the test compound as a compound for treatment or prevention of fibrosis,
wherein the substrate is prolyl 4-hydroxylase α subunit or a homolog thereof.

29. The method of claim 28, wherein either the synoviolin or the substrate is bound to a solid phase or comprises a label capable of binding to a solid phase.

30. The method of claim 28, wherein the prolyl 4-hydroxylase α subunit or a homolog thereof is any one of the following polypeptides (a) to (e):
(a) a polypeptide encoded by the nucleotide sequence of SEQ ID NO: 1;
(b) a polypeptide comprising the amino acid sequence of SEQ ID NO: 2;
(c) a polypeptide which comprises an amino acid sequence with a substitution, deletion, insertion, and/or addition of one or plural amino acids in the amino acid sequence of SEQ ID NO: 2 and can bind to synoviolin;
(d) a polypeptide which comprises an amino acid sequence encoded by a polynucleotide hybridizing under stringent conditions with a DNA comprising the nucleotide sequence of SEQ ID NO: 1 and can bind to synoviolin; and
(e) a polypeptide which comprises an amino acid sequence having a homology of 70% or more with the amino acid sequence of SEQ ID NO: 2 and can bind to synoviolin.

31. The method of claim 28, wherein the synoviolin or a homolog thereof is any one of the following polypeptides (A) to (E):
(A) a polypeptide encoded by the nucleotide sequence of SEQ ID NO: 3;
(B) a polypeptide comprising the amino acid sequence of SEQ ID NO: 4;
(C) a polypeptide which comprises an amino acid sequence with a substitution, deletion, insertion, and/or addition of one or plural amino acids in the amino acid sequence of SEQ ID NO: 4 and can bind to a polypeptide comprising the amino acid sequence of SEQ ID NO: 2;
(D) a polypeptide which hybridizes under stringent conditions with a DNA comprising the nucleotide sequence of SEQ ID NO: 3 and that can bind to a polypeptide comprising the amino acid sequence of SEQ ID NO: 2, and
(E) a polypeptide which comprises an amino acid sequence having a homology of 70% or more with the amino acid sequence of SEQ ID NO: 4 and can bind to a polypeptide comprising the amino acid sequence of SEQ ID NO: 2.

32. A pharmaceutical composition for treating and/or preventing fibrosis, comprising a compound selectable by the method of any one of claims 28 to 31 as an active ingredient.

33. A method of screening for a compound for treatment or prevention of rheumatoid arthritis, comprising the steps of:
a) incubating synoviolin or a homolog thereof and a substrate in the presence of a test compound under enabling conditions for substrate ubiquitination,
a') contacting either (i) a substrate or (ii) synoviolin or a homolog thereof with a test compound and incubating with the other of (i) and (ii) under enabling conditions for substrate ubiquitination, or
a") incubating a substrate and synoviolin or a homolog thereof under enabling conditions for substrate ubiquitination and contacting them with a test compound;
b) measuring level of substrate ubiquitination after any one of steps a), a'), and a"); and
c) selecting a test compound with a substrate ubiquitination level lower than a ubiquitination level measured in the absence of the test compound as a compound for treatment or prevention of rheumatoid arthritis,
wherein the substrate is prolyl 4-hydroxylase α subunit or a homolog thereof.

34. The method of claim 33, wherein the enabling conditions for substrate ubiquitination are provided by coexistence of the following components:
i) a ubiquitin activating enzyme;
ii) a ubiquitin transferase;
iii) ubiquitin; and
iv) adenosine triphosphate.

35. The method of claim 33, wherein the enabling conditions for substrate ubiquitination are provided by ubiquitinating the substrate in a cell expressing the substrate and the synoviolin or a homolog thereof.

36. The method of claim 33, wherein the level of substrate ubiquitination is measured using any one of the following levels as an index:
A) level of ubiquitinated substrate;
B) level of unubiquitinated substrate; and
C) level of prolyl 4-hydroxylase α subunit bioactivity.

37. The method of claim 33, wherein the prolyl 4-hydroxylase α subunit or a homolog thereof is any one of the following polypeptides (a) to (e):
(a) a polypeptide encoded by the nucleotide sequence of SEQ ID NO: 1;
(b) a polypeptide comprising the amino acid sequence of SEQ ID NO: 2;
(c) a polypeptide which comprises an amino acid sequence with a substitution, deletion, insertion, and/or addition of one or plural amino acids in the amino acid sequence of SEQ ID NO: 2 and can be ubiquitinated by synoviolin;
(d) a polypeptide which comprises an amino acid sequence encoded by a polynucleotide hybridizing under stringent conditions with a DNA comprising the nucleotide sequence of SEQ ID NO: 1 and can be ubiquitinated by synoviolin; and
(e) a polypeptide which comprises an amino acid sequence having a homology of 70% or more with the amino acid sequence of SEQ ID NO: 2 and can be ubiquitinated by synoviolin.

38. The method of claim 33, wherein the synoviolin or a homolog thereof is any one of the following polypeptides (A) to (E):
(A) a polypeptide encoded by the nucleotide sequence of SEQ ID NO: 3;
(B) a polypeptide comprising the amino acid sequence of SEQ ID NO: 4;
(C) a polypeptide which comprises an amino acid sequence with a substitution, deletion, insertion, and/or addition of one or plural amino acids in the amino acid sequence of SEQ ID NO: 4 and has an activity of ubiquitinating a polypeptide comprising the amino acid sequence of SEQ ID NO: 2;
(D) a polypeptide which hybridizes under stringent conditions with a DNA comprising the nucleotide sequence of SEQ ID NO: 3 and that has an activity of ubiquitinating a polypeptide comprising the amino acid sequence of SEQ ID NO: 2; and
(E) a polypeptide which comprises an amino acid sequence having a homology of 70% or more with the amino acid sequence of SEQ ID NO: 4 and has an activity of ubiquitinating a polypeptide comprising the amino acid sequence of SEQ ID NO: 2.

39. A pharmaceutical composition for treatment and/or prevention of rheumatoid arthritis, comprising a compound selectable by the method of any one of claims 33 to 38 as an active ingredient.

40. A kit for screening for a compound for treatment or prevention of rheumatoid arthritis, comprising the following components:
(1) synoviolin or a homolog thereof; and
(2) prolyl 4-hydroxylase α subunit or a homolog thereof.

41. The kit of claim 39, further comprising the following components:
(3) a ubiquitin activating enzyme;
(4) a ubiquitin transferase;
(5) ubiquitin; and
(6) adenosine triphosphate.

42. A kit for screening for a test compound for treatment or prevention of rheumatoid arthritis, comprising a cell expressing synoviolin or a homolog thereof, and prolyl 4-hydroxylase α subunit or a homolog thereof; and a means for measuring the ubiquitination level of the prolyl 4-hydroxylase α subunit or a homolog thereof.

43. A method of screening for a compound for treatment or prevention of rheumatoid arthritis, comprising the steps of:
a) incubating in the presence of a test compound under enabling conditions for binding of the synoviolin or a homolog thereof and the substrate;
a') contacting either (i) a substrate or (ii) synoviolin or a homolog thereof with a test compound and incubating under enabling conditions for binding of the synoviolin and the substrate, or
a") incubating under enabling conditions for binding of the substrate and the synoviolin or a homolog thereof and contacting them with a test compound;
b) measuring the level of binding between the substrate and the synoviolin or a homolog thereof after any one of steps a), a'), and a"); and
c) selecting a test compound with a binding level between the substrate and the synoviolin or a homolog thereof lower than a binding level measured in the absence of the test compound as a compound for treatment or prevention of rheumatoid arthritis,
wherein the substrate is prolyl 4-hydroxylase α subunit or a homolog thereof.

44. The method of claim 43, wherein either the synoviolin or the substrate is bound to a solid phase or comprises a label capable of binding to a solid phase.

45. The method of claim 43, wherein the prolyl 4-hydroxylase α subunit or a homolog thereof is any one of the following polypeptides (a) to (e):
(a) a polypeptide encoded by the nucleotide sequence of SEQ ID NO: 1;
(b) a polypeptide comprising the amino acid sequence of SEQ ID NO: 2;
(c) a polypeptide which comprises an amino acid sequence with a substitution, deletion, insertion, and/or addition of one or plural amino acids in the amino acid sequence of SEQ ID NO: 2 and can bind to synoviolin;
(d) a polypeptide which comprises an amino acid sequence encoded by a polynucleotide hybridizing under stringent conditions with a DNA comprising the nucleotide sequence of SEQ ID NO: 1 and can bind to synoviolin; and
(e) a polypeptide which comprises an amino acid sequence having a homology of 70% or more with the amino acid sequence of SEQ ID NO: 2 and can bind to synoviolin.

46. The method of claim 43, wherein the synoviolin or a homolog thereof is any one of the following polypeptides (A) to (E):
(A) a polypeptide encoded by the nucleotide sequence of SEQ ID NO: 3;
(B) a polypeptide comprising the amino acid sequence of SEQ ID NO: 4;
(C) a polypeptide which comprises an amino acid sequence with a substitution, deletion, insertion, and/or addition of one or plural amino acids in the amino acid sequence of SEQ ID NO: 4 and can bind to a polypeptide comprising the amino acid sequence of SEQ ID NO: 2;
(D) a polypeptide which hybridizes under stringent conditions with a DNA comprising the nucleotide sequence of SEQ ID NO: 3 and that can bind to a polypeptide comprising the amino acid sequence of SEQ ID NO: 2; and
(E) a polypeptide which comprises an amino acid sequence having a homology of 70% or more with the amino acid sequence of SEQ ID NO: 4 and can bind to a polypeptide comprising the amino acid sequence of SEQ ID NO: 2.

47. A pharmaceutical composition for treatment and/or prevention of rheumatoid arthritis, comprising a compound selectable by the method of any one of claims 43 to 46 as an active ingredient.

48. A polypeptide comprising the amino acid sequence of SEQ ID NO: 5.

49. A polypeptide comprising an amino acid sequence with a substitution, deletion, insertion, and/or addition of one or plural amino acids in the amino acid sequence of SEQ ID NO: 5,
wherein when the polypeptide is used as a test compound in the method of claim 1, the ubiquitination level of a substrate is lower than a ubiquitination level binding level measured in the absence of the polypeptide.

50. A polypeptide comprising an amino acid sequence with a substitution, deletion, insertion, and/or addition of one or plural amino acids in the amino acid sequence of SEQ ID NO: 5,
wherein when the polypeptide is used as a test compound in the method of claim 12, the level of binding between a substrate and synoviolin or a homolog thereof is lower than a binding level measured in the absence of the polypeptide.

51. A polynucleotide encoding the polypeptide of any one of claims 48 to 50.

52. A pharmaceutical composition for treatment and/or prevention of fibrosis, comprising the polypeptide of any one of claims 48 to 50 as an active ingredient.

53. A pharmaceutical composition for treatment and/or prevention of rheumatoid arthritis, comprising the polypeptide of any one of claims 48 to 50 as an active ingredient.


SEQUENCE LISTING

<110>  LOCOMOGENE INC.
       Eisai Co., Ltd.

<120>  METHOD OF DETECTING EFFECT OF CONTROLLING SYNOVIOLIN ACTIVITY

<130>  BHP-A0302P

<150>  JP 2003-295951
<151>  2003-08-20

<150>  JP 2003-295964
<151>  2003-08-20

<160>  5

<170>  PatentIn version 3.1

<210>  1
<211>  1605
<212>  DNA
<213>  Homo sapiens

<220>
<221>  CDS

<222>  (1)..(1605)

<223>

<400>  1

atg atc tgg tat ata tta att ata gga att ctg ctt ccc cag tct ttg      48

Met Ile Trp Tyr Ile Leu Ile Ile Gly Ile Leu Leu Pro Gln Ser Leu

1               5                   10                  15

gct cat cca ggc ttt ttt act tca att ggt cag atg act gat ttg atc      96

Ala His Pro Gly Phe Phe Thr Ser Ile Gly Gln Met Thr Asp Leu Ile

            20                  25                  30

cat act gag aaa gat ctg gtg act tct ctg aaa gat tat att aag gca      144

His Thr Glu Lys Asp Leu Val Thr Ser Leu Lys Asp Tyr Ile Lys Ala

        35                  40                  45

gaa gag gac aag tta gaa caa ata aaa aaa tgg gca gag aag tta gat      192

Glu Glu Asp Lys Leu Glu Gln Ile Lys Lys Trp Ala Glu Lys Leu Asp

    50                  55                  60

cgg cta act agt aca gcg aca aaa gat cca gaa gga ttt gtt ggg cat      240

Arg Leu Thr Ser Thr Ala Thr Lys Asp Pro Glu Gly Phe Val Gly His

65                  70                  75                  80

cca gta aat gca ttc aaa tta atg aaa cgt ctg aat act gag tgg agt      288

Pro Val Asn Ala Phe Lys Leu Met Lys Arg Leu Asn Thr Glu Trp Ser

                85                  90                  95

gag ttg gag aat ctg gtc ctt aag gat atg tca gat ggc ttt atc tct 336
Glu Leu Glu Asn Leu Val Leu Lys Asp Met Ser Asp Gly Phe Ile Ser
            100                 105                 110

aac cta acc att cag aga cag tac ttt cct aat gat gaa gat cag gtt 384
Asn Leu Thr Ile Gln Arg Gln Tyr Phe Pro Asn Asp Glu Asp Gln Val
        115                 120                 125

ggg gca gcc aaa gct ctg tta cgt ctc cag gat acc tac aat ttg gat 432
Gly Ala Ala Lys Ala Leu Leu Arg Leu Gln Asp Thr Tyr Asn Leu Asp
    130                 135                 140

aca gat acc atc tca aag ggt aat ctt cca gga gtg aaa cac aaa tct 480
Thr Asp Thr Ile Ser Lys Gly Asn Leu Pro Gly Val Lys His Lys Ser
145                 150                 155                 160

ttt cta acg gct gag gac tgc ttt gag ttg ggc aaa gtg gcc tat aca 528
Phe Leu Thr Ala Glu Asp Cys Phe Glu Leu Gly Lys Val Ala Tyr Thr
                165                 170                 175

gaa gca gat tat tac cat acg gaa ctg tgg atg gaa caa gcc cta agg 576
Glu Ala Asp Tyr Tyr His Thr Glu Leu Trp Met Glu Gln Ala Leu Arg
            180                 185                 190

caa ctg gat gaa ggc gag att tct acc ata gat aaa gtc tct gtt cta 624
Gln Leu Asp Glu Gly Glu Ile Ser Thr Ile Asp Lys Val Ser Val Leu
        195                 200                 205

gat tat ttg agc tat gcg gta tat cag cag gga gac ctg gat aag gca 672
Asp Tyr Leu Ser Tyr Ala Val Tyr Gln Gln Gly Asp Leu Asp Lys Ala
    210                 215                 220

ctt ttg ctc aca aag aag ctt ctt gaa cta gat cct gaa cat cag aga 720
Leu Leu Leu Thr Lys Lys Leu Leu Glu Leu Asp Pro Glu His Gln Arg
225                 230                 235                 240

gct aat ggt aac tta aaa tat ttt gag tat ata atg gct aaa gaa aaa 768
Ala Asn Gly Asn Leu Lys Tyr Phe Glu Tyr Ile Met Ala Lys Glu Lys
                245                 250                 255

gat gtc aat aag tct gct tca gat gac caa tct gat cag aaa act aca 816
Asp Val Asn Lys Ser Ala Ser Asp Asp Gln Ser Asp Gln Lys Thr Thr
            260                 265                 270

cca aag aaa aaa ggg gtt gct gtg gat tac ctg cca gag aga cag aag 864
Pro Lys Lys Lys Gly Val Ala Val Asp Tyr Leu Pro Glu Arg Gln Lys
        275                 280                 285

tac gaa atg ctg tgc cgt ggg gag ggt atc aaa atg acc cct cgg aga 912
Tyr Glu Met Leu Cys Arg Gly Glu Gly Ile Lys Met Thr Pro Arg Arg

290                 295                 300

cag aaa aaa ctc ttt tgc cgc tac cat gat gga aac cgt aat cct aaa     960
Gln Lys Lys Leu Phe Cys Arg Tyr His Asp Gly Asn Arg Asn Pro Lys
305                 310                 315                 320

ttt att ctg gct cca gct aaa cag gag gat gaa tgg gac aag cct cgt     1008
Phe Ile Leu Ala Pro Ala Lys Gln Glu Asp Glu Trp Asp Lys Pro Arg
                325                 330                 335

att att cgc ttc cat gat att att tct gat gca gaa att gaa atc gtc     1056
Ile Ile Arg Phe His Asp Ile Ile Ser Asp Ala Glu Ile Glu Ile Val
            340                 345                 350

aaa gac cta gca aaa cca agg ctg agc cga gct aca gta cat gac cct     1104
Lys Asp Leu Ala Lys Pro Arg Leu Ser Arg Ala Thr Val His Asp Pro
        355                 360                 365

gag act gga aaa ttg acc aca gca cag tac aga gta tct aag agt gcc     1152
Glu Thr Gly Lys Leu Thr Thr Ala Gln Tyr Arg Val Ser Lys Ser Ala
    370                 375                 380

tgg ctc tct ggc tat gaa aat cct gtg gtg tct cga att aat atg aga     1200
Trp Leu Ser Gly Tyr Glu Asn Pro Val Val Ser Arg Ile Asn Met Arg
385                 390                 395                 400

ata caa gat cta aca gga cta gat gtt tcc aca gca gag gaa tta cag 1248
Ile Gln Asp Leu Thr Gly Leu Asp Val Ser Thr Ala Glu Glu Leu Gln
                405                 410                 415

gta gca aat tat gga gtt gga gga cag tat gaa ccc cat ttt gac ttt 1296
Val Ala Asn Tyr Gly Val Gly Gly Gln Tyr Glu Pro His Phe Asp Phe
            420                 425                 430

gca cgg aaa gat gag cca gat gct ttc aaa gag ctg ggg aca gga aat 1344
Ala Arg Lys Asp Glu Pro Asp Ala Phe Lys Glu Leu Gly Thr Gly Asn
        435                 440                 445

aga att gct aca tgg ctg ttt tat atg agt gat gtg tct gca gga gga 1392
Arg Ile Ala Thr Trp Leu Phe Tyr Met Ser Asp Val Ser Ala Gly Gly
    450                 455                 460

gcc act gtt ttt cct gaa gtt gga gct agt gtt tgg ccc aaa aaa gga 1440
Ala Thr Val Phe Pro Glu Val Gly Ala Ser Val Trp Pro Lys Lys Gly
465                 470                 475                 480

act gct gtt ttc tgg tat aat ctg ttt gcc agt gga gaa gga gat tat 1488
Thr Ala Val Phe Trp Tyr Asn Leu Phe Ala Ser Gly Glu Gly Asp Tyr
                485                 490                 495

agt aca cgg cat gca gcc tgt cca gtg cta gtt ggc aac aaa tgg gta 1536
Ser Thr Arg His Ala Ala Cys Pro Val Leu Val Gly Asn Lys Trp Val

            500                 505                 510

tcc aat aaa tgg ctc cat gaa cgt gga caa gaa ttt cga aga cct tgt 1584
Ser Asn Lys Trp Leu His Glu Arg Gly Gln Glu Phe Arg Arg Pro Cys
        515                 520                 525

acg ttg tca gaa ttg gaa tga 1605
Thr Leu Ser Glu Leu Glu
    530

<210> 2
<211> 534
<212> PRT
<213> Homo sapiens

<400> 2
Met Ile Trp Tyr Ile Leu Ile Ile Gly Ile Leu Leu Pro Gln Ser Leu
1               5                   10                  15

Ala His Pro Gly Phe Phe Thr Ser Ile Gly Gln Met Thr Asp Leu Ile
            20                  25                  30

His Thr Glu Lys Asp Leu Val Thr Ser Leu Lys Asp Tyr Ile Lys Ala
        35                  40                  45

Glu Glu Asp Lys Leu Glu Gln Ile Lys Lys Trp Ala Glu Lys Leu Asp
    50                  55                  60

Arg Leu Thr Ser Thr Ala Thr Lys Asp Pro Glu Gly Phe Val Gly His
65                  70                  75                  80

Pro Val Asn Ala Phe Lys Leu Met Lys Arg Leu Asn Thr Glu Trp Ser
                85                  90                  95

Glu Leu Glu Asn Leu Val Leu Lys Asp Met Ser Asp Gly Phe Ile Ser
            100                 105                 110

Asn Leu Thr Ile Gln Arg Gln Tyr Phe Pro Asn Asp Glu Asp Gln Val
        115                 120                 125

Gly Ala Ala Lys Ala Leu Leu Arg Leu Gln Asp Thr Tyr Asn Leu Asp
    130                 135                 140

Thr Asp Thr Ile Ser Lys Gly Asn Leu Pro Gly Val Lys His Lys Ser
145                 150                 155                 160

Phe Leu Thr Ala Glu Asp Cys Phe Glu Leu Gly Lys Val Ala Tyr Thr
                165                 170                 175

Glu Ala Asp Tyr Tyr His Thr Glu Leu Trp Met Glu Gln Ala Leu Arg
            180                 185                 190

Gln Leu Asp Glu Gly Glu Ile Ser Thr Ile Asp Lys Val Ser Val Leu
        195                 200                 205
Asp Tyr Leu Ser Tyr Ala Val Tyr Gln Gln Gly Asp Leu Asp Lys Ala
    210                 215                 220
Leu Leu Leu Thr Lys Lys Leu Leu Glu Leu Asp Pro Glu His Gln Arg
225                 230                 235                 240
Ala Asn Gly Asn Leu Lys Tyr Phe Glu Tyr Ile Met Ala Lys Glu Lys
                245                 250                 255
Asp Val Asn Lys Ser Ala Ser Asp Asp Gln Ser Asp Gln Lys Thr Thr
            260                 265                 270
Pro Lys Lys Lys Gly Val Ala Val Asp Tyr Leu Pro Glu Arg Gln Lys
        275                 280                 285
Tyr Glu Met Leu Cys Arg Gly Glu Gly Ile Lys Met Thr Pro Arg Arg
    290                 295                 300
Gln Lys Lys Leu Phe Cys Arg Tyr His Asp Gly Asn Arg Asn Pro Lys
305                 310                 315                 320
Phe Ile Leu Ala Pro Ala Lys Gln Glu Asp Glu Trp Asp Lys Pro Arg

                325                 330                 335

Ile Ile Arg Phe His Asp Ile Ile Ser Asp Ala Glu Ile Glu Ile Val

            340                 345                 350

Lys Asp Leu Ala Lys Pro Arg Leu Ser Arg Ala Thr Val His Asp Pro

        355                 360                 365

Glu Thr Gly Lys Leu Thr Thr Ala Gln Tyr Arg Val Ser Lys Ser Ala

    370                 375                 380

Trp Leu Ser Gly Tyr Glu Asn Pro Val Val Ser Arg Ile Asn Met Arg

385                 390                 395                 400

Ile Gln Asp Leu Thr Gly Leu Asp Val Ser Thr Ala Glu Glu Leu Gln

                405                 410                 415

Val Ala Asn Tyr Gly Val Gly Gly Gln Tyr Glu Pro His Phe Asp Phe

            420                 425                 430

Ala Arg Lys Asp Glu Pro Asp Ala Phe Lys Glu Leu Gly Thr Gly Asn

        435                 440                 445

Arg Ile Ala Thr Trp Leu Phe Tyr Met Ser Asp Val Ser Ala Gly Gly

    450                 455                 460

Ala Thr Val Phe Pro Glu Val Gly Ala Ser Val Trp Pro Lys Lys Gly
465                 470                 475                 480

Thr Ala Val Phe Trp Tyr Asn Leu Phe Ala Ser Gly Glu Gly Asp Tyr
                485                 490                 495

Ser Thr Arg His Ala Ala Cys Pro Val Leu Val Gly Asn Lys Trp Val
            500                 505                 510

Ser Asn Lys Trp Leu His Glu Arg Gly Gln Glu Phe Arg Arg Pro Cys
        515                 520                 525

Thr Leu Ser Glu Leu Glu
    530

<210>  3
<211>  3374
<212>  DNA
<213>  Homo sapiens

<220>
<221>  CDS
<222>  (403)..(2256)
<223>

<400> 3
gccctttctt atgagcatgc ctgtgttggg ttgacagtga gggtaataat gacttgttgg 60
ttgattgtag atatagggct ctcccttgca aggtaattag gctccttaaa ttacctgtaa 120
gattttcttg ccacagcatc cattctggtt aggctggtga tcttctgagt agtgatagat 180
tggttggtgg tgaggtttac aggtgttccc ttctcttact cctggtgttg gctacaatca 240
ggtggcgtct agagcagcat gggacaggtg ggtaagggga gtcttctcat tatgcagaag 300
tgatcaactt aaatctctgt cagatctacc tttatgtagc ccggcagtcg cgcggattga 360
gcgggctcgc ggcgctgggt tcctggtctc cgggccaggg ca atg ttc cgc acg 414
gca gtg atg atg gcg gcc agc ctg gcg ctg acc ggg gct gtg gtg gct 462
cac gcc tac tac ctc aaa cac cag ttc tac ccc act gtg gtg tac ctg 510
acc aag tcc agc ccc agc atg gca gtc ctg tac atc cag gcc ttt gtc 558
ctt gtc ttc ctt ctg ggc aag gtg atg ggc aag gtg ttc ttt ggg caa 606
ctg agg gca gca gag atg gag cac ctt ctg gaa cgt tcc tgg tac gcc 654
gtc aca gag act tgt ctg gcc ttc acc gtt ttt cgg gat gac ttc agc 702
ccc cgc ttt gtt gca ctc ttc act ctt ctt ctc ttc ctc aaa tgt ttc 750
cac tgg ctg gct gag gac cgt gtg gac ttt atg gaa cgc agc ccc aac 798
atc tcc tgg ctc ttt cac tgc cgc att gtc tct ctt atg ttc ctc ctg 846
ggc atc ctg gac ttc ctc ttc gtc agc cac gcc tat cac agc atc ctg 894
acc cgt ggg gcc tct gtg cag ctg gtg ttt ggc ttt gag tat gcc atc 942
ctg atg acg atg gtg ctc acc atc ttc atc aag tat gtg ctg cac tcc 990
gtg gac ctc cag agt gag aac ccc tgg gac aac aag gct gtg tac atg 1038
ctc tac aca gag ctg ttt aca ggc ttc atc aag gtt ctg ctg tac atg 1086
gcc ttc atg acc atc atg atc aag gtg cac acc ttc cca ctc ttt gcc 1134
atc cgg ccc atg tac ctg gcc atg aga cag ttc aag aaa gct gtg aca 1182
gat gcc atc atg tct cgc cga gcc atc cgc aac atg aac acc ctg tat 1230
cca gat gcc acc cca gag gag ctc cag gca atg gac aat gtc tgc atc 1278
atc tgc cga gaa gag atg gtg act ggt gcc aag aga ctg ccc tgc aac 1326
cac att ttc cat acc agc tgc ctg cgc tcc tgg ttc cag cgg cag cag 1374
acc tgc ccc acc tgc cgt atg gat gtc ctt cgt gca tcg ctg cca gcg 1422
cag tca cca cca ccc ccg gag cct gcg gat cag ggg cca ccc cct gcc 1470
ccc cac ccc cca cca ctc ttg cct cag ccc ccc aac ttc ccc cag ggc 1518
ctc ctg cct cct ttt cct cca ggc atg ttc cca ctg tgg ccc ccc atg 1566
ggc ccc ttt cca cct gtc ccg cct ccc ccc agc tca gga gag gct gtg 1614
gct cct cca tcc acc agt gca gca gcc ctt tct cgg ccc agt gga gca 1662
gct aca acc aca gct gct ggc acc agt gct act gct gct tct gcc aca 1710
gca tct ggc cca ggc tct ggc tct gcc cca gag gct ggc cct gcc cct 1758
ggt ttc ccc ttc cct cct ccc tgg atg ggt atg ccc ctg cct cca ccc 1806
ttt gcc ttc ccc cca atg cct gtg ccc cct gcg ggc ttt gct ggg ctg 1854
acc cca gag gag cta cga gct ctg gag ggc cat gag cgg cag cac ctg 1902
gag gcc cgg ctg cag agc ctg cgt aac atc cac aca ctg ctg gac gcc 1950
gcc atg ctg cag atc aac cag tac ctc acc gtg ctg gcc tcc ttg ggg 1998
ccc ccc cgg cct gcc act tca gtc aac tcc act gag ggg act gcc act 2046
aca gtt gtt gct gct gcc tcc tcc acc agc atc cct agc tca gag gcc 2094
acg acc cca acc cca gga gcc tcc cca cca gcc cct gaa atg gaa agg 2142
cct cca gct cct gag tca gtg ggc aca gag gag atg cct gag gat gga 2190
gag ccc gat gca gca gag ctc cgc cgg cgc cgc ctg cag aag ctg gag 2238
tct cct gtt gcc cac tgacactgcc ccagcccagc cccagcctct gctcttttga 2293
gcagccctcg ctggaacatg tcctgccacc aagtgccagc tccctctctg tctgcaccag 2353
ggagtagtac ccccagctct gagaaagagg cggcatcccc taggccaagt ggaaagaggc 2413
tggggttccc atttgactcc agtcccaggc agccatgggg atctcgggtc agttccagcc 2473
ttcctctcca actcttcagc cctgtgttct gctggggcca tgaaggcaga aggtttagcc 2533
tctgagaagc cctcttcttc ccccacccct ttccaggaga aggggctgcc cctccaagcc 2593
ctacttgtat gtgcggagtc acactgcagt gccgaacagt attagctccc gttcccaagt 2653
gtggactcca gaggggctgg aggcaagcta tgaacttgct cgctggccca cccctaagac 2713
tggtacccat ttccttttct taccctgatc tccccagaag cctcttgtgg tggtggctgt 2773
gccccctatg ccctgtggca tttctgcgtc ttactggcaa ccacacaact cagggaaagg 2833
aatgcctggg agtgggggtg caggcgggca gcactgaggg accctgcccc gcccctcccc 2893
ccaggcccct ttcccctgca gcttctcaag tgagactgac ctgtctcacc cagcagccac 2953
tgcccagccg cactccaggc aagggccagt gcgcctgctc ctgaccactg caatcccagc 3013
gcccaaggaa ggccacttct caactggcag aacttctgaa gtttagaatt ggaattactt 3073
ccttactagt gtcttttggc ttaaattttg tcttttgaag ttgaatgctt aatcccggga 3133
aagaggaaca ggagtgccag actcctggtc tttccagttt agaaaaggct ctgtgccaag 3193
gagggaccac aggagctggg acctgcctgc ccctgtcctt tccccttggt tttgtgttac 3253
aagagttgtt ggagacagtt tcagatgatt atttaatttg taaatattgt acaaatttta 3313
atagcttaaa ttgtatatac agccaaataa aaacttgcat taacaaaaaa aaaaaaaaaa 3373
a 3374

<210> 4

<211> 617

<212> PRT

<213> Homo sapiens

<400> 4

<210> 5

<211> 21

<212>  PRT

<213>  Artificial

<400>  5

Asp Asn Val Cys Ile Ile Cys Arg Glu Glu Met Val Thr Gly Ala Lys

1               5                   10                  15

Arg Leu Asp Cys Asn

            20